# EUROPEAN PATENT APPLICATION

(11) **EP 3 957 721 A1**
(43) Date of publication of application: **23.02.2022**
(21) Application number: 20192024.6
(22) Date of filing: 20.08.2020
(51) Int. Cl.: C12N 5/079, A61K 35/30, C07K 14/705, C12N 15/85

(54) **RAPID PRODUCTION OF HUMAN NEURONAL CULTURES WITH SINGLE CELL RESOLUTION**

(71) Applicant: Life & Brain GmbH, 53127 Bonn (DE); Rheinische Friedrich-Wilhelms-Universität Bonn, 53113 Bonn (DE)
(72) Inventor: Stappert, Laura, 53127 Bonn (DE); Klaus, Frederike, 53113 Bonn (DE); Brüstle, Oliver, 53127 Bonn (DE)
(74) Representative: Zwicker, Jörk

(57) **Abstract**

The present invention provides a method of converting one or more neural cells (NCs) into one or more induced neuronal cells, comprising (a) providing NCs; and (b) contacting the NCs with a transcription factor (TF) or expressing the TF within the NCs, wherein the TF comprises a DNA binding moiety and a transactivation domain (TAD), wherein the DNA binding moiety specifically binds to a direct repeat with zero spacing (DR0) response element; thereby producing induced neuronal cells. The invention further provides methods of converting one or more neural cells (NCs) into one or more induced neuronal cells by inhibiting the expression of GCNF in the NCs or expressing a GCNF target gene in the NCs or contacting the NCs with the gene product of a GCNF target gene. The present invention also provides a TF comprising a DNA binding moiety specifically binding to a direct repeat with zero spacing (DR0) response element and a TAD, a nucleic acid comprising said TF, a vector comprising said nucleic acid and a cell comprising said TF. In addition, the invention provides said cell for use in a method of treatment or prevention of a neurological condition as well as the use of said cell in a method of drug screening or as a disease model.

## Description

The present invention relates to methods of converting neural cells, in particular human neural stem cells, into neuronal cells.

### BACKGROUND OF THE INVENTION

Human neurons generated from human pluripotent stem cells (hPSCs) provide a unique platform to identify disease-causing mechanisms and to perform drug screening approaches in the context of human brain disorders. While there are many protocols to generate human neural cell types by directed differentiation of hPSCs, these "run-through protocols" have limited utility in high throughput screening (HTS) campaigns, since they entail repeated labor-intensive procedures, are time-consuming and usually yield a heterogeneous population of cells. The efficiency and robustness of such protocols can be increased by applying a combination of small molecules to mimic signaling cues that are relevant during brain development or by overexpressing pro-neuronal transcription factors (e.g. ASCL1 or NGN2) to accelerate neuronal fate acquisition. The latter approach was designated as "forward programming" or "direct neuronal conversion" of hPSCs (Pawlowski et al., 2017) and has been implemented at several laboratories. Another approach to reduce the time and variability in generating human neurons is to use hPSC-derived neural stem cells (NSCs) as intermediate cell source instead of initiating the differentiation process each time from hPSCs. Nevertheless, even when starting from established NSC lines, it still takes several weeks to generate human neurons. Neuronal differentiation from hPSC-derived NSCs can be induced by withdrawing the growth factors driving NSC self-renewal and be further accelerated by applying the γ-secretase inhibitor DAPT to block Notch signaling. Another approach is to induce neuronal differentiation of hPSC-derived NSCs by overexpression of the pro-neuronal transcription factor NGN2, in analogy to the NGN2-based "forward programming" approach of hPSCs. However, in the protocols developed by Ho et al., 2016 and Cheng et al., 2017 additional treatment with the anti-mitotic drug Ara-C to deplete unwanted, remaining proliferative cells is required. Another problem is that NSCs tend to differentiate in clusters of densely packed cells, which poses a significant challenge for high-content imaging (HCI) based assays. In fact, most induced neuronal preparations cannot be directly used for image-based read-outs but instead require additional manipulation steps, such as re-plating the neurons to achieve a density appropriate for HCI or anti-mitotic treatment to eliminate unwanted proliferative cells. While this cluster formation may resemble at some degree neural tissue self-organization, it also makes it difficult to identify and reliably extract information of individual cells within the clusters for image-based analysis. Re-plating of differentiated neurons onto microplates for imaging is labor-intensive and might be associated with substantial cell loss, jeopardizing the results of the read-out.

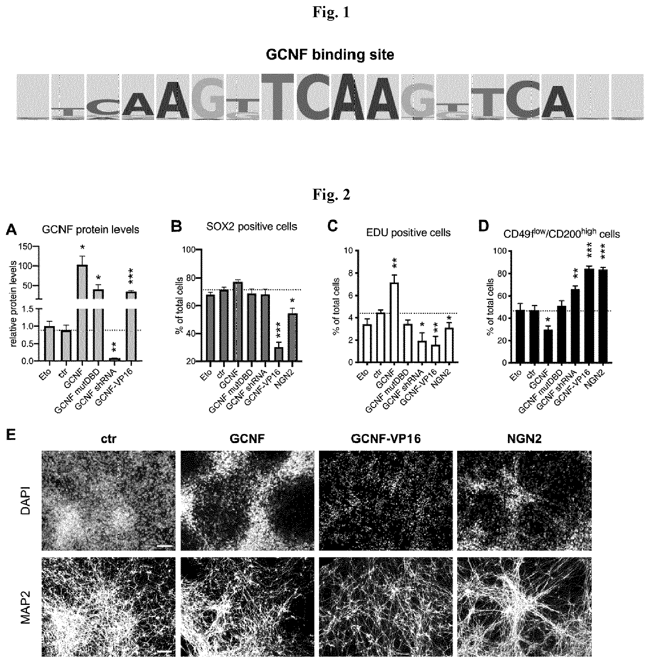

GCNF (Germ Cell Nuclear Factor, official gene symbol NR6A1) belongs to the nuclear receptor family of ligand-dependent transcription factors (reviewed by Wagner and Cooney, 2013). Since no cognate endogenous ligand binding to GCNF has been identified so far, it is considered to be an orphan nuclear receptor. GCNF binds to a specific DR0 nuclear receptor response element, which comprises a direct repeat of the core motif AG(G/T)TCA with zero spacing (Figure 1). GCNF comprises its own unique subfamily within the superfamily of nuclear receptors with a DNA-binding domain that resembles retinoid X receptor (RXR) and a ligand-binding domain related to COUP transcription factor 2. Lacking the canonical activation-function-2 domain within the ligand-binding domain, GCNF is considered to act as a repressor of target gene expression (Cooney et al., 1998). GCNF exerts its repressive function in part by competing with other nuclear receptors and transcription activators, such as ERRα, SF-1 (NR5A1), LRH-1 (NR5A2) and CREMτ, by interacting with several co-repressors, such as NCoR and SMRT, and by recruiting DNA methyltransferase to the promoter of target genes to induce promoter methylation and silencing. GCNF is known for its role during exit from pluripotency, where it inhibits expression of Oct4 (POU5F1) and other pluripotency genes, as well as for its function as regulator of germ cell development (reviewed by Wang and Cooney, 2013, *supra;* Zechel, 2005). GCNF is expressed in the developing CNS of mice and frogs, and depletion of GCNF leads to a failure of neural tube closure among other developmental defects (reviewed by Wang and Cooney, 2013, *supra*)*.* Knock-down of GCNF during retinoic acid-induced differentiation of mouse embryonic carcinoma cells (a surrogate of PSCs) impaired the generation of neurons, whereas overexpression of GCNF promoted neuronal differentiation (Koziollek-Drechsler et al., 2005; Sattler et al., 2004) pointing to a positive effect of GCNF on neuronal differentiation.

Surprisingly, the inventors have found that the expression of a GCNF-transactivator in neural progenitor cells (NPCs) results in accelerated neuronal differentiation of these cells. The present invention is based on the discovery that expression of a transcription factor (TF) comprising a DNA-binding domain (DBD) specifically binding to a DR0 response element and a strong transactivation domain (TAD) accelerates and synchronizes neuronal differentiation of NPCs. The invention provides for the generation of induced neurons from neural cells, wherein the method according to the invention is characterized *inter alia* by the following advantages:
(i) the method is faster than the methods known in the state of the art and enables generation of neuronal cultures from NSCs/NPCs within 48 hours;
(ii) the method does not require the use of anti-mitotic drugs;
(iii) the cultures have a homogenous cell density;
(iv) the cultures are free of cell clusters, show a reduced number of clusters or a reduced size of clusters ;
(v) the cultures are suitable for high-content imaging (HCI) based assays;
(vi) the method requires fewer steps than the methods known in the state of the art to generate cultures suitable for HCI based assays;
(vii) the method allows for the generation of large amounts of induced neuronal cells from cryopreserved NSCs/NPCs;
(viii) the method is less expensive than the methods known in the state of the art;
(ix) the method is more reliable and easier to reproduce than the methods known in the state of the art;
(x) the cultures are suitable for CNS disease modelling and/or drug screening approaches.

### SUMMARY OF THE INVENTION

In a first aspect, the present invention relates to a method of converting one or more neural cells (NCs) into one or more induced neuronal cells, comprising (a) providing NCs; and (b) contacting the NCs with a transcription factor (TF) or expressing the TF within the NCs, wherein the TF comprises a DNA binding moiety and a transactivation domain (TAD), wherein the DNA binding moiety specifically binds to a direct repeat with zero spacing (DRO) response element; thereby producing induced neuronal cells.

In a second aspect, the present invention relates to a method of converting one or more NCs into one or more induced neuronal cells, comprising (a) providing NCs; and (b) inhibiting the expression of GCNF in the NCs; thereby producing induced neuronal cells.

In a third aspect, the present invention relates to a method of converting one or more NCs into one or more induced neuronal cells, comprising (a) providing NCs; and (b) expressing a GCNF target gene in the NCs or contacting the NCs with the gene product of a GCNF target gene; thereby producing induced neuronal cells.

In a fourth aspect, the present invention relates to a TF comprising a DNA binding moiety and a heterologous TAD, wherein the DNA binding moiety specifically binds to a direct repeat with zero spacing (DRO) response element.

In a fifth aspect, the present invention relates to a nucleic acid encoding the TF of the fourth aspect of the invention.

In a sixth aspect, the present invention relates to a vector comprising the nucleic acid of the fifth aspect of the invention, wherein in particular the vector is selected from the group consisting of a lentiviral vector, an adenoviral vector, an adenovirus associated viral (AAV) vector and a sendai virus vector.

In a seventh aspect, the present invention relates to a cell comprising the TF of the fourth aspect, the nucleic acid of the fifth aspect and/or the vector of the sixth aspect of the invention.

In an eighth aspect, the present invention relates to a cell produced by the method of the first, second or third aspect of the invention.

In a ninth aspect, the present invention relates to a microplate comprising the cell of the seventh or eighth aspect of the invention.

In a tenth aspect, the present invention relates to the use of a cell of the seventh or eighth aspect of the invention in a method of drug screening.

In an eleventh aspect, the present invention relates to the use of a cell of the seventh or eighth aspect of the invention as a disease model.

In a twelfth aspect, the present invention relates to the cell of the seventh or eighth aspect of the invention for use in a method of treatment or prevention of a neurological condition, in particular neuronal damage, neuronal loss or a neurodegenerative disease.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention, which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Klbl, H. eds. (1995), Helvetica Chimica Acta, CH-4010 Basel, Switzerland) and as described in "Pharmaceutical Substances: Syntheses, Patents, Applications" by Axel Kleemann and Jurgen Engel, Thieme Medical Publishing, 1999; the "Merck Index: An Encyclopedia of Chemicals, Drugs, and Biologicals", edited by Susan Budavari et al., CRC Press, 1996, and the United States Pharmacopeia-25/National Formulary-20, published by the United States Pharmcopeial Convention, Inc., Rockville Md., 2001.

Throughout this specification and the claims which follow, unless the context requires otherwise, the word "comprise", and variations such as "comprises" and "comprising", will be understood to imply the inclusion of a stated feature, integer or step or group of features, integers or steps but not the exclusion of any other feature, integer or step or group of integers or steps.

As used in this specification and the appended claims, the singular forms "a", "an", and "the" include plural referents, unless the content clearly dictates otherwise.

Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

To practice the present invention, unless otherwise indicated, conventional methods of chemistry, biochemistry, and recombinant DNA techniques are employed which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

In the following, some definitions of terms frequently used in this specification are provided. These terms will, in each instance of its use, in the remainder of the specification have the respectively defined meaning and preferred meanings.

The terms "polynucleotide" and "nucleic acid" are used interchangeably herein and are understood as a polymeric or oligomeric macromolecule made from nucleotide monomers. Nucleotide monomers are composed of a nucleobase, a five-carbon sugar (such as but not limited to ribose or 2'-deoxyribose), and one to three phosphate groups. Typically, a nucleic acid is formed through phosphodiester bonds between the individual nucleotide monomers. In the context of the present invention preferred nucleic acid molecules include but are not limited to ribonucleic acid (RNA), modified RNA, deoxyribonucleic acid (DNA), and mixtures thereof such as e.g. RNA-DNA hybrids. The nucleic acids, can e.g. be synthesized chemically, e.g. in accordance with the phosphotriester method (see, for example, Uhlmann, E. & Peyman, A. (1990) Chemical Reviews, 90, 543-584).

In the context of the present invention, the term "somatic cell" encompasses any cell in an organism that cannot give rise to all types of cells in an organism, i.e. it is not pluripotent. In other words, somatic cells are cells that have differentiated sufficiently that they will not naturally generate cells of all three germ layers of the body, i.e. ectoderm, mesoderm and endoderm.

In the context of the present invention, the term "pluripotent" refers to cells with the ability to give rise to progeny that can undergo differentiation, under appropriate conditions, into cell types that collectively exhibit characteristics associated with cell lineages from the three germ layers (endoderm, mesoderm, and ectoderm). A "stem cell' is a cell characterized by the ability of self-renewal through mitotic cell division and the potential to differentiate into specialized stem cells or somatic cells of a tissue or an organ. Among mammalian stem cells, embryonic and somatic stem cells may be distinguished. Pluripotent stem cells, which include embryonic stem cells, embryonic germ cells and induced pluripotent cells, can contribute to tissues of a prenatal, postnatal or adult organism. Somatic stem cells (also known as adult stem cells or tissue stem cells) are rare populations of undifferentiated cells that are found in the body, are capable of self-renewal and may give rise to a limited number of mature cell types that build the tissue in which they reside.

In the context of the present invention, the term "primary cells" is used to refer to cells and cell cultures that have been derived from a subject and allowed to grow *in vitro* for a limited number of passages, i.e. passaging of the culture.

In the context of the present invention, the term "neural stem cell (NSC)" refers to a cell capable of (1) generating differentiated neural cells (neurons, astrocytes and oligodendrocytes) and (2) having some capacity for self-renewal, and being able to give rise to differentiated progeny in addition to themselves through asymmetric cell division (reviewed by Gage, 2000). In the context of the present invention, the term "neural progenitor/precursor cell (NPC)" refers to a cell that can also generate neural progeny but has only limited self-renewal capacity. NSCs/NPCs express some of the phenotypic markers that are characteristic of the neural lineage as described in Martínez-Cerdeño and Noctor, 2018 and Zhang and Jiao, 2015. Typically, they do not produce progeny of other embryonic germ layers when cultured by themselves *in vitro,* unless dedifferentiated or reprogrammed in some fashion. NPCs are present in the CNS of developing embryos but are also found in the neonatal and mature adult brain. NPCs are characterized based on their location in the brain, morphology, gene expression profile, temporal distribution and function. NPCs can be generated *in vitro* by differentiating ESCs or iPSCs using different protocols (reviewed by Karus et al., 2014; reviewed by Conti and Cattaneo, 2010). When they can be kept as stable proliferating population these hES/hiPSCs-derived NPCs are often referred to as NSC line.

In the context of the present invention, the term "induced neuronal cell" is meant to refer to cells of the neuronal lineage i.e. neuronal precursor cells and mature neurons that arise from a neural progenitor/precursor cell by experimental manipulation.

As used herein, the term "protein", "peptide", "polypeptide", "peptides" and "polypeptides" are used interchangeably throughout. These terms are used in the context of the present invention to refer to both naturally occurring peptides, e.g. naturally occurring proteins and synthesized peptides that may include naturally or non-naturally occurring amino acids.

As used herein, the term "complement" is used to refer to a nucleic acid that is capable of base-pairing with another nucleic acid according to the standard Watson-Crick, Hoogsteen or reverse Hoogsteen binding complementarity rules. As used herein "another nucleic acid" may refer to a separate molecule or a spatial separated sequence of the same molecule. In certain embodiments, the term "complement" refers to a nucleic acid that is capable of hybridizing to another nucleic acid strand or duplex under stringent conditions, as would be understood by one of ordinary skill in the art.

As used herein the term "transcription factor" refers to a protein, protein complex or protein RNA complex that specifically binds to a DNA element, typically a double stranded DNA element within the genome of a cell that activates or represses transcription of that gene upon binding to the DNA element. Transcription factors generally comprise the DNA binding activity in a domain or moiety that is separate from the domain that activates or represses transcription. A domain that activates transcription is generally referred to as transactivation domain (TAD) and a domain that represses transcription is generally referred to as transrepression domain. Often the DNA binding domain or moiety can be separated from the regulatory domain (RD) and both maintain their respective functional activity. This property allows the creation of fusion proteins in which the DNA binding domain of a first transcription factor is fused to the RD of a second transcription factor and *vice versa* thereby creating a new hybrid transcription factor that imparts, e.g. the DNA binding activities of the first transcription factor to the RD of the second transcription factor. It is immediately apparent to the skilled person that such hybrid transcription factors can lead to altered transcription of those genes, which transcription is controlled by the first transcription factor. As used herein the term "transcription factor" also encompasses synthetic transcription factors based on clustered regularly interspaced short palindromic repeat (CRISPR) proteins (CRISPR-based transcription factors). The generation of CRISPR-based transcription factors is reviewed in Pandelakis et al., 2020. Shortly, catalytically dead Cas9 (dCas9) proteins are fused to a transactivation or transrepression domain. The dCas9 associates with a guideRNA, which can recognize and hybridize with a complementary sequence in the genomic DNA. The complex of dCas9 and guide RNA thus forms a "DNA binding moiety" as defined below. It is generally believed that transcription factors exert their transcriptional control function by recruiting further proteins and protein complexes to the transcriptional control elements of a gene. The factors that may be recruited comprise chromatin remodelers that condense or loosen the chromatin structure at or around the promoter or enhancer region of a gene or by recruiting components of the basal transcriptional machinery, e.g. TF_{II}D to the core promoter of a gene.

As used herein the term "DNA binding moiety" refers to that part of a transcription factor that is necessary and sufficient for specific binding of that transcription factor to a DNA element within a regulatory sequence of a gene. In the case of transcription factors that are proteins or protein complexes, the DNA binding moiety is referred to as DNA binding domain (DBD). In the case of CRISPR-based transcription factors, the DNA binding moiety is a complex of dCas9 and guide RNA.

As used herein the term "DNA binding domain (DBD)" refers to a DNA binding moiety of a transcription factor that is a proteins or protein complex. Thus, a DBD is a polypeptide domain. Since the majority of transcription factors specifically bind to DNA elements as dimers, i.e. dimerization is a prerequisite for specific DNA binding, the DBD typically comprises a dimerization domain that either leads to the formation of homodimers or heterodimers. Accordingly, typically the DBD of a transcription factor comprises a domain that specifically interacts with nucleotides of the DNA element, i.e. the DNA interaction domain, and a domain that provides an interaction surface for a second transcription factor molecule, i.e. the dimerization domain. Due to the formation of dimers most DNA elements comprise repeat elements wherein one repeat is bound by one and the other repeat element by the other protein. Exemplary DBDs comprise winged-helix like DNA binding domains, basic helix-loop-helix (bHLH) domain, the helix-turn-helix (HTH) domain or the leucine zipper (LZ) domain.

The term "transactivation domain" refers to that part of a transcription factor that is necessary and sufficient to enhance the transcription of a gene once the transcription factor is bound to a DNA element within a regulatory sequence of that gene. The skilled person is well aware how to delineate a transactivation domain within a given transcription factor. Typically, the transactivation domain can be delineated by Ala scanning of the protein and by N-terminal and C-terminal deletion analysis.

As used herein, the term "heterologous" is a relative term, which when used with reference to portions of a nucleic acid indicates that the nucleic acid comprises two or more subsequences that are not found in the same relationship to each other in nature. For instance, a nucleic acid that is recombinantly produced typically has two or more sequences from unrelated genes synthetically arranged to make a new functional nucleic acid, e.g., a promoter from one source and a coding region from another source. The two nucleic acids are thus heterologous to each other in this context. When added to a cell, the recombinant nucleic acids would also be heterologous to the endogenous genes of the cell. Similarly, a heterologous protein indicates that the protein comprises two or more subsequences that are not found in the same relationship to each other in nature (e.g., a "fusion protein" where the two subsequences are encoded by a single nucleic acid sequence).

As used herein, the term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (naturally occurring) form of the cell or express a second copy of a native gene.

As used herein, "treat", "treating", "treatment" or "therapy" of a disease or disorder means accomplishing one or more of the following: (a) reducing the severity of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in an individual that has previously had the disorder(s); and (e) limiting or preventing recurrence of symptoms in individuals that were previously symptomatic for the disorder(s).

In the following, the aspects of the present invention will be described in more detail. These aspects are listed with specific embodiments, however, it should be understood that the specific embodiments disclosed in the context of one aspect may be combined in any manner and in any number with the other aspects and are also disclosed herein as additional embodiments of the other aspects. The variously described examples and preferred embodiments should not be construed to limit the present invention to only the explicitly described embodiments. This description should be understood to support and encompass embodiments, which combine the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a method of converting one or more neural cells (NCs) into one or more induced neuronal cells, comprising (a) providing NCs; and (b) contacting the NCs with a transcription factor (TF) or expressing the TF within the NCs, wherein the TF comprises a DNA binding moiety and a transactivation domain (TAD), wherein the DNA binding moiety specifically binds to a direct repeat with zero spacing (DRO) response element; thereby producing induced neuronal cells. The TF of the first aspect of the invention binds to DR0 response elements comprised in transcription control elements of genes of the NCs. While the binding of, e.g. GCNF to such elements will repress the transcription of the respective gene, the binding of the TF of the first aspect of the invention will lead to the activation of transcription of such genes due to the presence of a TAD within the TF.

The TF used in the method of the present invention is preferably a hybrid transcription factor comprising a TAD and DNA binding moiety that are heterologous to each other. More preferably, the TF used in the method of the present invention is a hybrid transcription factor comprising a TAD and DBD that are heterologous to each other, i.e. a fusion protein of a DNA binding domain of a first transcription factor and of a transactivation domain of a second transcription factor or of a heterologous protein domain that has transactivating activity. The resulting TF, thus has the DNA binding specificity of the first transcription factor and the transactivation activity of the second transcription factor. Transcription factors are often homodimers. In this case the contacting of the cell with one TF is sufficient to provide the cell with the homodimeric and, thus functional TF. The skilled person is aware that recombinantly expressed GCNF in COS1 cells forms a homo-dimer, whereas endogenously presented GCNF in P19 cells acts as a homo-oligomer forming the transiently RA-induced factor TRIF (Gu et al., 2005c). Some transcription factors are, however, heterodimers. In this case the expression of the TF will lead to the formation of a heterodimer comprising the natural dimerization partner and the TF. In this case the cell may also be contacted with two different TFs wherein the DNA binding domains of the heterodimeric transcription factors are each separately fused to a TAD.

The DR0 response element is comprised in the transcription control elements of various cellular genes. In preferred embodiments, the DR0 response element to which the DBD of the TF binds specifically comprises or consists of the following nucleotide sequence:
N₁N₂N₃N₄N₅AAGN₆N₇N₈N₉ (SEQ ID NO: 21)
wherein N₁ is selected from A, C and T, N₂ is selected from G, T, and A, N₃ is selected from G and T, N₄ is selected from T and C, N₅ is selected from C and T, N₆ is selected from G and T, N₇ is selected from T, C, G and A, N₈ is selected from C, A, G and T, and N₉ is selected from A, T, G and C. In a preferred embodiment N₁ is A, and/or N₂ is G, and/or N₃ is selected from G and T, and/or N₄ is selected from T, and/or N₅ is C, and/or N₆ is selected from G and T, and/or N₇ is selected from T, and C, and/or N₈ is selected from C, and A, and N₉ is selected from A. In a particular embodiment the DR0 response element comprises or consists of the following nucleotide sequence AGkTCAAGkTCA (SEQ ID NO: 1) and its complement, wherein k is in each case independently selected from G or T. The skilled person is aware that the indicated DR0 sequence is a consensus sequence and that the actual binding sites present in the promoter region of target genes may slightly differ. Regardless, also these sequences will be bound by a DNA binding moiety as defined in the present invention, i.e. a DNA binding moiety that specifically binds to the indicated DR0 element comprising or consisting of SEQ ID NO: 1. In other words, the TF according to the invention binds to the indicated DR0 response element, but will also bind to a sequence that slightly differs from that sequence, e.g. AGGCCAAGTTCA (SEQ ID NO: 9), AGTTCAAGGTAA (SEQ ID NO: 10), TGGTCAAGTACT (SEQ ID NO: 11), CAGCCAAGGTCA (SEQ ID NO: 12), AGTTTAAGGCCA (SEQ ID NO: 13), ATGTCAAGGTCA (SEQ ID NO: 14), AGGTCAAGGCTA (SEQ ID NO: 15), AGTTCAAGGTCA (SEQ ID NO: 16), AGGTCAAGTTCC (SEQ ID NO: 17), AGGTCAAGGCGA (SEQ ID NO: 18), AGTTCAAGGCCA (SEQ ID NO: 19) (Gu et al., 2005a; Gu et al., 2005b), AGTTCAAGTGCG (SEQ ID NO: 20). The skilled person is well aware of how to test the ability and strength of the binding of the TFs used in the method of the present invention to the DR0 response elements noted above. Such methods include gel shift analysis, DNA footprinting and binding assays in which either the TF or the DR0 response element is immobilized to a surface (Surface plasmon resonance).

In preferred embodiments, the DNA binding moiety further binds to a cAMP response element / nuclear receptor binding site (CRE/NR) composite site. In preferred embodiments, the CRE/NR composite site is a dual DNA response element for the binding of cAMP response element modulator tau (CREMτ) and GCNF. In preferred embodiments, the CRE/NR composite site comprises or consists of the following nucleotide sequence: TGACGTCA (SEQ ID NO: 22).

In some embodiments, the TF is a synthetic CRISPR-based transcription factor. It can be envisioned that such a synthetic CRISPR-based transcription factor comprises a guideRNA comprising a targeting sequence against DR0 according to SEQ ID NO: 23 (UAGCCUUGACCU) or SEQ ID NO: 32 (GUAGCCUUGACCU C). In addition to the targeting sequence, the guide RNA further comprises scaffold sequences. The skilled person is aware that guideRNAs comprising truncated (short) targeting sequences are sufficient for transcriptional regulation in a dCas9-based approach (Kiani et al., 2015). Thus, a guideRNA comprising a targeting sequence according to SEQ ID NO: 23 or SEQ ID NO: 32 is capable of targeting the DR0 motif, in particular SEQ ID NO: 15. An exemplary guide RNA for use in the method according to the first aspect of the invention comprising a truncated targeting sequence against DR0 is the guide RNA according to SEQ ID NO: 33.

This guideRNA forms a complex with dCas9 and guides the synthetic CRISPR-based TF to DR0 response elements. Consecutively, the TAD of the synthetic CRISPR-based TF activates transcription of DR0 regulated genes.

In preferred embodiments, the TF is a protein or protein complex and the DNA binding moiety is a DBD, i.e. a DNA binding polypeptide domain. In preferred embodiments, the DBD comprises or consists of a DBD of a TF selected from SF1/NR5A1, LRH-1/NR5A2 and Germ Cell Nuclear Factor (GCNF/NR6A1), preferably human SF1/NRSA1, LRH-1/NR5A2 or GCNF/NR6A1. The skilled person is aware that SF1/NR5A1, LRH-1/NR5A2 and GCNF/NR6A1 all bind to DR0 response elements (Gu et al., 2005a; Gu et al., 2005b; Yang et al., 2007; Sung et al., 2012; Park et al., 2017). In preferred embodiments, the DBD comprises or consists of a DBD of GCNF. In more preferred embodiments, the DBD comprises or consists of a DBD of human GCNF. In certain embodiments, the DBD comprising or consisting of a DBD of SF1/NR5A1, LRH-1/NR5A2 or GCNF/NR6A1 optionally comprises amino acid modifications that do not abolish binding of the DBD to DR0 response elements or that enhance binding to DR0 response elements. It is preferred that the DNA binding part of the DBD is mutated with respect to the naturally occurring protein to increase the strength of the binding to the DR0 response elements. If modified in this way the TF binds stronger to the DR0 response elements in the genome of the NCs and more efficiently replaces the naturally occurring transcription factor from which the DBD is derived from the DR0 response elements. Preferably, such a mutated DBD has one, two or three amino acid substitutions in the DNA interaction domain of the transcription factor on which the DBD is based and a binding affinity that is at least two-fold increased in comparison to the transcription factor on which the DBD is based.

It is possible that TFs that naturally form homo - or heterodimers will homo - or heterodimerize with their dimerization partner naturally occurring in the NCs. Thus the generated TF may not be as effective in transactivating genes comprising DR0 response elements in their promoter or enhancers as a homo - or heterodimers in which both molecules comprise a TAD. To improve the pairing of homo or heterodimerizing TFs, it is preferred to modify the dimerization domain of the DBD to favour dimerization among the TFs used in the present invention.

In preferred embodiments, the DBD comprises or consists of SEQ ID NO: 2. Alternatively, the DBD comprises or consists of a sequence having at least 90%, at least 95%, at least 97% or at least 99% identity to SEQ ID NO: 2 and exhibits at least the same binding to DR0 response elements as the DBD comprising or consisting of SEQ ID NO: 2. In even more preferred embodiments, the DBD comprises or consists of SEQ ID NO: 3 or SEQ ID NO: 4, most preferably SEQ ID NO: 4. Alternatively, the DBD comprises or consists of a sequence having at least 90%, at least 95%, at least 97% or at least 99% identity to SEQ ID NO: 3 or 4 and exhibits at least the same binding to DR0 response elements as the DBD comprising or consisting of SEQ ID NO: 3 or 4.

In addition to the DNA binding moiety, the TF according to the invention comprises a TAD that is capable of activating transcription of a gene if recruited to the regulatory DNA sequences of said gene by the DNA binding moiety. The DR0 response elements bound by the DNA binding moiety may be located in promoter sequences, intergenic regions or genic regions. In preferred embodiments, the DR0 response element is located 8 kb upstream to 1 kb downstream of the transcription start site (TSS). In preferred embodiments, the DR0 response element is located 5 kb, 3 kb, 2 kb, 1.5 kb, 1 kb or 0.5 kb upstream to 1 kb downstream of the TSS. In preferred embodiments, the DR0 response element is located between 3 kb, 2 kb, 1.5 kb, 1 kb or 0.5 kb upstream of the TSS and the TSS. In preferred embodiments, the TAD induces unfolding of chromatin structure, recruitment of a transcription initiation complex, initiation of RNA transcription and/or elongation of RNA transcription.

In preferred embodiments, the TAD comprises or consists of a TAD selected from the group consisting of the activation domain of a nuclear receptor, in particular of a steroid, thyroid or retinoid acid receptor; a synthetic or chimeric activation domain; a basic or acidic amino acid activation domain; a viral activation domain; an activation domain of the nine-amino-acid TAD (9aaTAD) family, in particular a 9aaTAD as comprised in Gal4, Oaf1, Pip2, Pdr1, Pdr3, Leu3, Teal, Pho4, Gln3, Gcn4, Msn2, Msn4, Rtg3, CREB, CREBaB6, E2A, MLL, p53-TADI, p53-TADII, FOXO3, NF-kB, NFAT, CEBPA/E, ESX, ELF3, ETV1, KLF2/4, EBNA2, VP16, HSF1, HSF2, HsfA, Gli3, Sox18, PIF, Dreb2a, MTF1, OREB1, WRKY45, NS1, MKL1, VP16, EBNA2, KBP220, ECapLL, P201, AH, or B42; a glutamine-rich activation domain, in particular as present in POU2F1 (Oct1), POU2F2 (Oct2) and Sp1); a proline-rich activation domain, in particular as present in c-jun, AP2 or Oct2; an isoleucine-rich activation domain, in particular as present in NTF-1; a Tat activation domain; a BP64 activation domain; a TAF-1 activation domain; a TAF-2 activation domain; a TAU-1 activation domain; a TAU-2 activation domain; a YAP activation domain, a bHLH protein activation domain, in particular as present in MYOD, ASCL1 or NGN2, or a fragment of said activation domains or a modified version thereof.

In preferred embodiments, the TAD comprises or consists of the TAD of VP16, VP64, YAP or MYOD. In more preferred embodiments, the TAD comprises or consists of the TAD of VP16 or VP64. The TAD of VP16 and VP64 are closely related, wherein the TAD of VP64 corresponds to 4x the TAD of VP16.

In even more preferred embodiments, the TAD comprises or consists of the TAD of VP16. VP16 entails a strong TAD that facilitates DNA transcription by inducing chromatin unfolding and recruiting components of the transcription pre-initiation complex.

In preferred embodiments, the TAD comprises or consists of SEQ ID NO: 5. Alternatively, the TAD comprises or consists of a sequence having at least 90%, at least 95%, at least 97% or at least 99% identity to SEQ ID NO: 5 and exhibits at least the same transactivation activity as the TAD comprising or consisting of SEQ ID NO: 5. In even more preferred embodiments, the TAD comprises or consists of SEQ ID NO: 6 or SEQ ID NO: 7. Alternatively, the TAD comprises or consists of a sequence having at least 90%, at least 95%, at least 97% or at least 99% identity to SEQ ID NO: 7 and exhibits at least the same transactivation activity as the TAD comprising or consisting of SEQ ID NO: 7.

In preferred embodiments, the TF comprises or consists of the amino acid sequence of SEQ ID NO: 8.

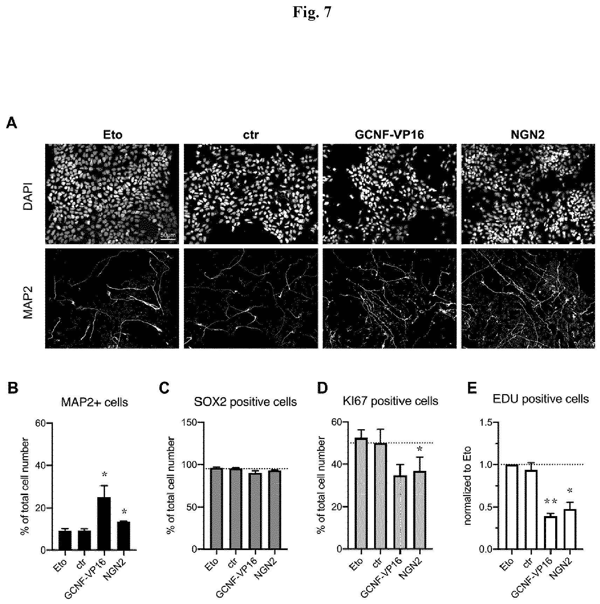

The inventors discovered that a DR0 response element-binding transactivator, in particular a transactivator comprising the DBD of human GCNF, is able to induce neuronal differentiation of hPSC-derived NSCs. GCNF-VP16 acts as artificial pro-neuronal factor driving hPSC-NSC towards neuronal differentiation within 48 hours (Figure 7).

The invention is not limited to a full-length GCNF-VP16 fusion protein, and derivatives of GCNF or any other polypeptide domain that confers binding to the DR0 element may be fused with a variety of transcriptional activation domains (N-terminally or C-terminally) known in the art. This includes GCNF DBD from animal species, mutated versions of the GCNF DBD to increase DNA binding affinity, e.g. Gly149Arg and Pro151Arg (Weikum et al., 2016), and related DBDs from other nuclear factors known to bind to DR0 motifs, e.g. SF1/NR5A1 and LRH-1/NR5A2. This also includes GCNF derivatives, which lack the ligand binding domain (LBD), since it was shown that binding to DNA and target regulation is independent from the presence of the LBD (Okumura et al., 2013). This may also include other strategies to interfere with the endogenous function by GCNF, e.g. GCNF knock-down strategies (including siRNA) or expression of a dominant-negative GCNF.

In preferred embodiments, the NCs are pre-directed towards the neural lineage. Any cell that is pre-directed towards the neural lineage could be used as host cell to generate neurons by means of overexpressing a DR0 response element-binding transactivator, in particular a GCNF-VP16 transactivator.

In preferred embodiments, the NCs are neural stem cells (NSCs) or neural progenitor cells (NPCs), preferably human NSCs or human NPCs. In certain embodiments, the human NSCs or human NPCs are human embryonic stem (hES) cell-derived NPCs/NSCs, human induced pluripotent cell (hiPSC)-derived NPCs/NSCs, transient NPCs generated by neural induction of hPSCs, induced neural stem cells (iNSCs) generated from fibroblasts or blood cells, primary human neural progenitor cells; or a cell line with neurogenic differentiation capacity, in particular a cell line selected from L-Myc, ReNcell CX, LUHMES, N2TD2 and SH-SY5Y.

In preferred embodiments, the NCs are human NSCs generated from a human induced pluripotent stem cell (hiPSC-NSCs).

Methods for the generation of preferred hES/hiPSC-derived NPCs/NSCs are described in Koch et al., 2009, Li et al., 2011, Reinhardt et al., 2013, Gorris et al., 2015 or Roese-Körner et al., 2016. These cells include NSCs/NPCs referred to as long-term neural epithelial-like stem cells (lt-NES), primitive neural stem cells (pNSCs), small molecule neural precursor cells (smNPCs), and radial glia-like NPCs (RGL-NPCs).

Methods for the generation of transient NPCs by neural induction of hPSCs are described in Elkabetz et al., 2008; Shi et al., 2012 and Fasano et al., 2010. These cells include cells referred to as rosette-forming NSCs (R-NSCs), neocortical stem/progenitor cells and floor plate progenitor cells.

Methods for the generation of iNSCs generated from fibroblasts or blood cells are described in Kumar et al., 2012; Mitchell et al., 2014; Thier et al., 2019; Lee et al., 2015 and Sheng et al., 2018.

Primary human neural progenitor cells can be retrieved as described in the literature (Darbinyan et al., 2013, Pollard et al., 2006) or bought from commercial providers (e.g. StemPro NSCs (ThermoFischer Scientific), Poietics^{™} Neural Progenitor Cells (NHNP) (Lonza)).

In certain embodiments, step b of the method according to the first aspect of the invention is effected by contacting the NCs with the TF. In these instances, the TF (also referred to as "GCNF-transactivator") is provided as a nuclear acting polypeptide. To promote transport of the polypeptide across the cell membrane, polypeptide sequence may be fused to a cell-permeable peptide. A number of cell-permeable peptides are known in the art and include penetratin (derived from the third alpha helix of Drosophila melanogaster transcription factor Antennapaedia) and tat-based peptides (derived from HIV-1 tat basic region amino acid sequence). Other permeant domains include poly-arginine motifs, for example, the region of amino acids 34-56 of HIV-1 rev protein, nona-arginine, octa-arginine, and the like (see, for example, Futaki et al., 2003; Wender et al., 2000). The nona-arginine sequence is particularly efficient (Uemura et al., 2002). The polypeptide may be isolated and purified in accordance with conventional methods of recombinant synthesis. A lysate may be prepared of the expression host cell and the lysate purified using HPLC, exclusion chromatography, gel electrophoresis, affinity chromatography, or other purification technique. For the most part, the compositions which are used will comprise at least 20% by weight of the desired product, more usually at least about 75% by weight, preferably at least about 95% by weight, and for therapeutic purposes, usually at least about 99.5% by weight, in relation to contaminants related to the method of preparation of the product and its purification. Usually, the percentages will be based upon total protein. Polypeptides may be produced recombinantly not only directly, but also as a fusion polypeptide with additional heterologous polypeptides, e.g. a polypeptide having a specific cleavage site at the N-terminus of the mature protein or polypeptide. Expression vectors usually contain a selection gene, also termed a selectable marker. This gene encodes a protein necessary for the survival or growth of transformed host cells grown in a selective culture medium. The polypeptide may also be prepared by cell-free protein synthesis (CFPS) using automated protein synthesizer known in the art. Various commercial synthetic apparatuses are available, for example, automated synthesizers by Applied Biosystems, Inc., Beckman, etc. By using synthesizers, naturally occurring amino acids may be substituted with unnatural amino acids. The particular sequence and the manner of preparation will be determined by convenience, economics, purity required, and the like. Other methods of preparing polypeptides in a cell-free system include, for example, those methods taught in U.S. Application Ser. No. 61/271,000. Following purification by commonly known methods in the art, GCNF-transactivator polypeptides are provided to the subject cells by standard protein transduction methods. In some cases, the protein transduction method includes contacting cells with a composition containing a carrier agent and the polypeptide. Examples of suitable carrier agents and methods for their use include, but are not limited to, commercially available reagents such as Chariot^{™} (Active Motif, Inc., Carlsbad, Calif.) described in U.S. Pat. No. 6,841,535; Bioport^{™} (Gene Therapy Systems, Inc., San Diego, Calif.), GenomeONE (Cosmo Bio Co., Ltd., Tokyo, Japan), and ProteoJuice^{™} (Novagen, Madison, Wis.), or nanoparticle protein transduction reagents as described in, e.g., U.S. patent application Ser. No. 10/138,593.

In preferred embodiments, step b of the method according to the first aspect of the invention is effected by expressing the TF within the NCs.

The expression may be effected by transiently or stably introducing a nucleic acid encoding the transcription factor into the NCs. Alternatively or in addition, the expression may be effected by inducing in the NCs the transcription of the TF from a nucleic acid that has been previously introduced into the NCs. The skilled person is aware that introduction of a nucleic acid into NCs may also be effected by introduction of the nucleic acid into cells from which the NCs have been obtained, preferably pluripotent stem cells. If the nucleic acid encoding the TF that has been introduced into the NCs further comprises an inducible promoter that controls the expression of the TF, induction of the expression of the TF can be effected by contacting the NCs with a compound activating the inducible promoter. Inducible promoters have been extensively reviewed in the prior art and the skilled person is well capable of choosing a suitable inducible promotor to carry out the method according to the invention. Examples of inducible promoters are promoters that can be induced by tetracycline, doxycycline, cumate, rapamycin, tamoxifen, FKCsA, which is a heterodimer of FK506 and cyclosporin A or absisic acid. E preferred example of an inducible promoter in a doxycycline-inducible promoter, i.e. a promoter that is induced in the presence of doxycycline. In preferred embodiments, the nucleic acid encoding the TF and further comprising an inducible promoter controlling the expression of the TF is stably introduced into pluripotent stem cells from which NCs are generated. During or after, preferably after the neural differentiation, expression of the TF is induced by treating the NCs with a compound activating the inducible promoter, e.g. doxycycline.

Various techniques known in the art may be used to introduce nucleic acids into the target cells, e.g. electroporation, calcium precipitated DNA, fusion, transfection, lipofection, viral infection and the like.

The nucleic acids may be delivered in plasmids or minicircle DNAs, which will be maintained episomally.

Nucleic acids may be also delivered as RNAs, e.g. by using synthetic modified mRNAs. There are various techniques to produce RNA molecules by in vitro-transcription reaction or by direct chemical synthesis know to the art. RNAs may comprise modifications that confer stability to the mRNAs or decrease mRNA immunogenicity. The RNA may be formulated in a liposomal transfer vehicle to facilitate delivery to the host cell. The transfection medium may also contain an immunosuppressant to reduce immunogenicity when transfecting cells with RNA. The following synthetic modifications may be used to protect the RNAs from cellular defenses against ssRNA: phosphatase treatment, substituting cytidine with 5-methylcytidine, and/or substituting uridine with pseudouridine.

In preferred embodiments, a nucleic acid encoding the TF is permanently introduced into the NCs. This can be effected by integration of a nucleic acid encoding the TF into the genome of the NC, in particular by lentiviral-based integration or integration into a safe harbor locus via genome editing using site-specific endonucleases, in particular zinc-finger nucleases, meganucleases, transcription activator-like effector (TALE) nucleases or CRISPR-associated (Cas9) nuclease.

The nucleic acid may be provided to the subject cells via a virus. In other words, the cells are contacted with viral particles comprising the GCNF-transactivator nucleic acids. Retroviruses, for example, lentiviruses, are particularly suitable to such methods. In certain embodiments, the stable integration into the genome of the NC is achieved by lentiviral-based integration. The present invention describes the use of the LentiX Tet-On Advanced Inducible Expression System for lentiviral-based gene transfer of a doxycycline-inducible expression system into host cells. Methods of introducing the lentiviral vectors comprising nucleic acids into packaging cell lines suitable to produce viral particles that can infect mammalian cells and methods of collecting the viral particles that are generated by the packaging lines are well known in the art. Commonly used lentiviral vectors are "replication deficient", i.e. unable to produce viral proteins required for productive infection. The component necessary for producing lentiviral particles are split across multiple plasmids, which entail (1) a lentiviral transfer plasmid encoding the nucleic acids of interest to be delivered into the target cells, (2) packaging plasmids and (3) an envelope plasmid. Production of lentiviral particles are usually created in a transient transfection system, in which a packaging cell line is transfected with the different plasmid outlined above and the nucleic acid of interest is packaged into viral capsids by the packaging cell line. The LentiX system that is used in the present invention is based on the HIV-derived "2^{nd} generation packaging system", which was developed in Didier Trono's lab. Other non-limiting examples of virus vectors that may be used to deliver a nucleic acid to host cells include adenoviral vectors, adeno-associated viral (AAV) vectors and Sendai viral vectors.

In alternative embodiments, stable integration into the genome of the NC comprises integration into a safe harbor locus via genome editing using site-specific endonucleases. As an alternative to the described lentiviral-based integration of the doxycycline-inducible GCNF-VP16 expression cassette into hPSC-derived NSCs (see Example 1 and 2), the same cassette may be introduced into a genomic safe harbor locus via genome editing of hPSCs (Qian et al., 2014). In contrast to the random integration of lentiviral vectors, which might affect gene expression by insertional mutagenesis, targeted genome editing in safe harbor loci avoids unpredicted interactions with the host genome. Safe harbor loci of the human genome, which are used most frequently, are the adeno-associated virus site 1 (AAVS1), the chemokine (C-C motif) receptor 5 (CCR5) and the human reverse oriented splice acceptor clone 26 (ROSA26) locus (Irion et al., 2007; Papapetrou and Schambach, 2016). Stable integration of the transgene in the safe harbor locus is facilitated by homologous directed repair mechanism of the host cells following DNA double strand breaks, which were introduced by site specific endonucleases to the predetermined genomic safe harbor locus. For this purpose, different site-specific endonucleases can be used. Suitable site-specific endonucleases are zinc-finger nucleases, meganucleases, transcription activator-like effector (TALE) nucleases or CRISPR-associated (Cas9) nuclease (Papapetrou and Schambach, 2016).

In preferred embodiments, the nucleic acid is under transcriptional control of a promoter. Vectors used for providing GCNF-transactivator nucleic acid will typically comprise suitable promoters for driving the expression, that is, transcriptional activation, of the nucleic acids. This may include ubiquitously acting promoters, for example the CMV-promoter and the EFla promoter, or inducible promoters that are active in particular cell populations or that respond to the presence of drugs, such as tetracycline and derivates (doxycycline). The vectors may also contain a fluorescent reporter or a selectable marker (e.g. genes that confer resistance to various selection drugs or genes that compensate for an essential gene that is defective in the cell line to be transfected) so that transfected cells can be enriched by virtue of the fluorescent reporter expression or by culturing the cells in appropriate selective medium.

In most preferred embodiments, expression of the transcription factor is effected by stably introducing a nucleic acid encoding the TF into the genome of the NCs or the cells from which the NCs are obtained, wherein most preferably the nucleic acid encoding the TF is under the control of an inducible promoter, e.g. a doxycycline-inducible promoter.

The transcription factor according to the first aspect of the invention has a transactivation activity of at least 2-fold, in particular at least 10-fold induction of the expression of a gene selected from the group consisting of ZIC3, NFIB, BCL11A, NEUROD1, and NEUROG2. In the context of the present specification, the expression "at least 2-fold induction of the expression of a gene" is meant to indicate that the expression of a gene is increased at least by factor 2. The data showing induction of gene expression by method of the invention using the TF according to the invention is presented in Table 1 below.

**Table 1**

| **Gene Symbol** | **Fold Change** | **P-val** | **FDR P-val** | Nervous System/ Neuronal | Axon/ Synapse/ Dendrite | Ion transport/ Channel/ NT | Adhesion | Migration | Proliferation |
|---|---|---|---|---|---|---|---|---|---|
| **# *Genes*** | | | | ***200*** | ***120*** | ***77*** | ***129*** | ***75*** | ***97*** |
| ZIC3 | 223,77 | 7,28E-18 | 7,80E-14 | N | | | | | |
| NFIB | 147,36 | 9,27E-19 | 1,99E-14 | N | ASD | | | | P |
| NR6A1 | 78,44 | 1,74E-14 | 5,33E-11 | | | | | | P |
| BCL11A | 48,38 | 1,04E-16 | 7,47E-13 | N | ASD | | | | |
| HCN1 | 39,9 | 3,30E-09 | 8,04E-07 | | | ICN | | | |
| MCF2 | 29,3 | 2,69E-09 | 6,92E-07 | | ASD | | | | |
| MYBPC1 | 24,88 | 7,35E-06 | 3,00E-04 | | | | A | | |
| SEMA3A | 24,3 | 5,19E-07 | 3,59E-05 | N | ASD | | | M | |
| CDH12 | 23,21 | 1,94E-07 | 1,77E-05 | | | | A | | |
| MPPED2 | 22,04 | 3,00E-08 | 3,99E-06 | N | | | | | |
| CNTN1 | 21,97 | 2,00E-07 | 1,78E-05 | N | | ICN | A | | |
| CD9 | 14,57 | 9,00E-04 | 1,16E-02 | | | | A | | P |
| CDK15 | 13,65 | 6,09E-06 | 3,00E-04 | | | | | | P |
| NTSR1 | 13,59 | 3,62E-08 | 4,65E-06 | N | | | | | |
| FLRT2 | 12,83 | 2,30E-12 | 3,52E-09 | N | ASD | | A | M | |
| CBLN1 | 12,82 | 1,31E-08 | 2,18E-06 | N | ASD | | A | | |
| GAS7 | 12,17 | 4,02E-07 | 2,98E-05 | N | | | | | P |
| NYAP2 | 12,03 | 6,94E-09 | 1,34E-06 | N | | | | | |
| RYR3 | 11,17 | 1,00E-06 | 6,12E-05 | | | ICN | | | |
| BRINP1 | 11,14 | 2,36E-10 | 1,23E-07 | N | | | | | P |
| CBLN2 | 11,04 | 4,03E-09 | 9,25E-07 | | ASD | | | | |
| FOXP2 | 10,95 | 1,38E-10 | 8,00E-08 | | | | | | P |
| CHL1 | 10,16 | 1,68E-09 | 5,14E-07 | N | ASD | | A | M | |
| GRM5 | 9,64 | 5,95E-09 | 1,19E-06 | N | | | | | |
| PCDH10 | 9,34 | 1,10E-08 | 1,87E-06 | | | | A | | |
| PIEZO2 | 9,11 | 3,00E-04 | 4,80E-03 | | | ICN | | | |
| BCL2L11 | 8,93 | 1,52E-06 | 8,52E-05 | N | | | A | | P |
| TGFB3 | 8,78 | 8,84E-06 | 3,00E-04 | N | | | | | P |
| GPR139 | 8,74 | 1,70E-05 | 6.00E-04 | N | | | | | |
| ADARB1 | 8,49 | 4,23E-12 | 5,58E-09 | | | | | M | P |
| ATP2B2 | 8,46 | 1,58E-08 | 2,50E-06 | N | ASD | ICN | | | |
| PPFIA2 | 8,38 | 1,20E-08 | 2,01E-06 | N | | ICN | A | | |
| BARHL2 | 8,25 | 1,44E-05 | 5.00E-04 | N | ASD | | | M | |
| KCNMA1 | 8,19 | 1,31E-10 | 8,00E-08 | | | ICN | | | |
| DSCAML1 | 8,18 | 1.64E-10 | 9,28E-08 | N | ASD | | A | | |
| SLITRK3 | 8,13 | 3,17E-14 | 7,54E-11 | | ASD | | | | |
| FSTL4 | 7,79 | 4,05E-11 | 3,47E-08 | N | | | | | |
| GFRA2 | 7,65 | 2,08E-07 | 1,82E-05 | N | | | | | |
| EPHA5 | 7,59 | 2,99E-09 | 7,56E-07 | N | ASD | | | | |
| SEMA3A | 7,46 | 8,63E-10 | 3,08E-07 | N | ASD | | | M | |
| CNTN4 | 7,29 | 2,89E-08 | 3,90E-06 | N | ASD | | A | | |
| ROBO2 | 7,22 | 2,06E-08 | 3,07E-06 | N | ASD | | A | | |
| SGK1 | 7,19 | 4,50E-07 | 3,20E-05 | N | | ICN | | M | P |
| DPP4 | 6,97 | 6,00E-04 | 8,90E-03 | | | | A | M | P |
| SIK1 | 6,94 | 2,57E-06 | 1,00E-04 | | | ICN | | | P |
| IL1RAPL1 | 6,93 | 1,22E-12 | 2,19E-09 | N | ASD | | A | | |
| ADORA1 | 6,4 | 1,47E-11 | 1,50E-08 | N | | ICN | | M | P |
| AP3B2 | 6,39 | 1,08E-09 | 3,66E-07 | | ASD | | | | |
| CAMK1D | 6,31 | 3,78E-08 | 4.79E-06 | N | ASD | | | | |
| SPRY1 | 6,11 | 4,37E-06 | 2,00E-04 | N | | | | | P |
| PCLO | 6,09 | 1,14E-08 | 1,93E-06 | | ASD | | | | |
| DLG2 | 5,73 | 4,26E-10 | 1,90E-07 | N | ASD | | | | |
| ADGRL1 | 5,61 | 3,86E-09 | 9,01 E-07 | | ASD | | A | | |
| SH3GL3 | 5,5 | 5,67E-09 | 1,15E-06 | N | | | | | |
| AHR | 5,49 | 1,38E-09 | 4,41 E-07 | | | | | | P |
| CNTNAP3B | 5,48 | 5,25E-08 | 6,16E-06 | | | | A | | |
| PRKCG | 5,47 | 4,27E-07 | 3,11E-05 | N | | | | | |
| AKAP9 | 5,42 | 6,95E-09 | 1,34E-06 | | | | | | P |
| NR4A3 | 5,39 | 8,29E-07 | 5,32E-05 | N | ASD | | | | P |
| SLC4A10 | 5,34 | 5,13E-09 | 1,09E-06 | N | | ICN | | | |
| BCL11B | 5,29 | 1,45E-09 | 4,57E-07 | N | ASD | | | | P |
| NKAIN2 | 5,29 | 2,71E-09 | 6,92E-07 | | | ICN | | | |
| SHB | 5,15 | 4,03E-07 | 2,98E-05 | | | | | | P |
| SNAP25 | 5,03 | 8,16E-06 | 3,00E-04 | N | | ICN | | | |
| CTNNA2 | 4,9 | 4,33E-08 | 5,42E-06 | | ASD | | A | M | |
| MYT1 L | 4,83 | 4,10E-09 | 9,25E-07 | N | | | | | |
| KCNA3 | 4,7 | 1,38E-07 | 1,31E-05 | | | ICN | | | |
| CNTNAP3 | 4,67 | 8,46E-08 | 9,09E-06 | | | | A | | |
| SLC6A17 | 4,56 | 6,50E-06 | 3,00E-04 | N | | ICN | | | |
| PTPRM | 4,52 | 1,82E-07 | 1,67E-05 | N | ASD | | A | M | P |
| PLPPR1 | 4,5 | 9,45E-08 | 9,79E-06 | N | | | | | |
| SHTN1 | 4,49 | 9,67E-13 | 1,89E-09 | N | ASD | | A | M | |
| FOXP1 | 4,47 | 2,65E-08 | 3,65E-06 | N | ASD | | | M | P |
| PTPRR | 4,44 | 8,00E-04 | 1,08E-02 | | | | A | M | |
| RPS6KA5 | 4,44 | 2,52E-05 | 8,00E-04 | | ASD | | | | |
| KCNQ3 | 4,4 | 9,88E-06 | 4,00E-04 | | | ICN | | | |
| LRFN5 | 4,36 | 1,32E-06 | 7,72E-05 | | ASD | | | | |
| RAB3A | 4,36 | 8,41 E-08 | 9,09E-06 | N | ASD | ICN | | | |
| VASH2 | 4,25 | 2,07E-09 | 6,0OE-07 | | | | | | P |
| KIFAP3 | 4,21 | 3,42E-09 | 8,23E-07 | | | | A | | P |
| CHRM2 | 4,2 | 4,89E-08 | 5.95E-06 | N | | | | | |
| NRSN1 | 4,2 | 1,77E-05 | 6,00E-04 | N | | | | | |
| SDC3 | 4,14 | 3,20E-06 | 2,00E-04 | | | | | M | |
| CDH18 | 4,09 | 3,49E-06 | 2,00E-04 | | | | A | | |
| SLC24A4 | 4,08 | 6,72E-06 | 3,00E-04 | | | ICN | | | |
| DMTN | 4,01 | 7,68E-08 | 8,40E-06 | | | | A | M | |
| MAB21L1 | 4 | 7,30E-03 | 5,62E-02 | | | | | | P |
| GRIK5 | 3,99 | 1,51E-06 | 8,49E-05 | N | | | | | |
| DNM3 | 3,95 | 4,78E-05 | 1,30E-03 | | ASD | | | | |
| RIT2 | 3,93 | 4,32E-07 | 3,11E-05 | N | | | | | |
| HPGD | 3,92 | 8,56E-08 | 9,13E-06 | | | | | | P |
| PITX2 | 3,91 | 2,00E-04 | 4,50E-03 | N | | | | M | P |
| GFRA1 | 3,89 | 1,66E-09 | 5,14E-07 | N | | | | | |
| SLC9A9 | 3,87 | 1,00E-04 | 3,00E-03 | | | ICN | | | |
| ADCY1 | 3,86 | 8,54E-05 | 2,00E-03 | | ASD | | | | |
| PLPPR5 | 3,86 | 9,73E-06 | 4,00E-04 | N | | | | | |
| C3orf58 | 3,85 | 8,11E-09 | 1,50E-06 | | | | | | P |
| ANK3 | 3,83 | 3,09E-09 | 7,68E-07 | N | ASD | ICN | A | | |
| CALCRL | 3,82 | 7,37E-05 | 1,70E-03 | | | ICN | | | P |
| RCVRN | 3,81 | 1,00E-03 | 1,32E-02 | | | ICN | | | |
| NRG1 | 3,8 | 9,53E-07 | 5,89E-05 | N | ASD | ICN | A | M | P |
| TUBA4A | 3,76 | 1,35E-06 | 7,81 E-05 | | | | | | P |
| NRXN1 | 3,71 | 6,80E-06 | 3,00E-04 | N | ASD | ICN | A | | |
| CADM2 | 3,68 | 3,12E-09 | 7,68E-07 | | | | A | | |
| DOCK10 | 3,68 | 3,84E-06 | 2,00E-04 | | | | | M | |
| ATCAY | 3,63 | 1,47E-06 | 8,33E-05 | N | | | | | |
| AGAP2 | 3,62 | 1,52E-06 | 8,52E-05 | N | | | | | P |
| PTPRO | 3,57 | 4,23E-07 | 3,08E-05 | N | ASD | | A | | |
| PTGFR | 3,56 | 8,74E-07 | 5,48E-05 | | | | | | P |
| CNTNAP2 | 3,49 | 2,83E-07 | 2,30E-05 | N | ASD | | A | | |
| PCDH7 | 3,47 | 3,11E-05 | 9,00E-04 | | | | A | | |
| FEZ1 | 3,46 | 7,28E-06 | 3,00E-04 | N | ASD | | A | | |
| CACNB1 | 3,44 | 4,19E-06 | 2,00E-04 | N | | | | | |
| NANOS1 | 3,44 | 4,67E-05 | 1,20E-03 | N | | | | M | |
| NEUROG2 | 3,36 | 1,96E-07 | 1,78E-05 | N | | | | | |
| FAIM2 | 3,35 | 4,70E-03 | 4,09E-02 | N | | | | | |
| SEMA3A | 3,34 | 2,95E-05 | 9,00E-04 | N | ASD | | | M | |
| TNIK | 3,34 | 3,84E-07 | 2,88E-05 | | ASD | | | | |
| PCDH9 | 3,33 | 4,40E-07 | 3,16E-05 | | | | A | | |
| BSN | 3,32 | 2,05E-07 | 1,80E-05 | | ASD | | | | |
| SLC4A4 | 3,3 | 3,98E-05 | 1,10E-03 | | | ICN | | | |
| SCN1A | 3,29 | 6,95E-07 | 4,59E-05 | N | | ICN | | | |
| DSCAM | 3,25 | 2,34E-05 | 7,00E-04 | N | ASD | | A | | |
| NEUROD6 | 3,24 | 1,00E-04 | 2,70E-03 | N | | | | | |
| CDON | 3,21 | 2,44E-05 | 8,00E-04 | N | | | A | | P |
| LRRTM2 | 3,21 | 8,00E-04 | 1,06E-02 | | ASD | | | | |
| APBA1 | 3,2 | 6,13E-07 | 4,12E-05 | N | | ICN | A | | |
| CDK5R1 | 3,2 | 1,65E-06 | 9,09E-05 | N | ASD | | A | M | P |
| DCLK2 | 3,19 | 4,45E-10 | 1,91E-07 | N | | | | | |
| ADGRG1 | 3,18 | 1,00E-04 | 2,80E-03 | N | | | A | M | P |
| AJAP1 | 3,18 | 1,79E-07 | 1,65E-05 | | | | A | | |
| ZMIZ1 | 3,18 | 1,80E-03 | 2,03E-02 | | | | | | P |
| KCNK9 | 3,17 | 6,78E-09 | 1,34E-06 | | | ICN | | | |
| MYT1L | 3,16 | 4,00E-04 | 6,60E-03 | N | | | | | |
| A TP13A2 | 3,15 | 5,10E-03 | 4,32E-02 | N | | ICN | | | |
| VAV3 | 3,15 | 3,29E-06 | 2,00E-04 | | | | A | | P |
| PRKCE | 3,14 | 2,26E-08 | 3,24E-06 | | | | A | M | P |
| CACHD1 | 3,13 | 5,63E-06 | 2,00E-04 | | | ICN | | | |
| CUX2 | 3,11 | 1,10E-07 | 1,10E-05 | | ASD | | | | |
| HCN4 | 3,1 | 4,00E-04 | 6,20E-03 | | | ICN | | | |
| GRIN1 | 3,09 | 4,44E-06 | 2,00E-04 | N | ASD | ICN | | | |
| TIGAR | 3,09 | 6,70E-03 | 5,28E-02 | N | | | | | |
| KIAA2022 | 3,08 | 8,64E-07 | 5,45E-05 | N | | | | | |
| SERPINI1 | 3,06 | 2,58E-07 | 2,14E-05 | N | | | A | | |
| ERBB4 | 3,05 | 3,65E-05 | 1,00E-03 | N | | | | M | P |
| KALRN | 3,05 | 2,12E-06 | 1,00E-04 | N | | | | M | P |
| DCLK1 | 3,03 | 4,19E-08 | 5,28E-06 | N | ASD | | | M | |
| DOC2B | 3,03 | 2,44E-07 | 2,05E-05 | N | | ICN | | | |
| DMD | 3,02 | 2,59E-05 | 8,00E-04 | N | | ICN | A | | |
| KCNN1 | 3,02 | 9,40E-08 | 9,78E-06 | | | ICN | | | |
| SCRT1 | 3,02 | 3,00E-04 | 5,70E-03 | N | | | | M | |
| PTPRD | 3,01 | 4,92E-09 | 1,08E-06 | N | ASD | | A | | |
| TSHZ3 | 2,99 | 7,70E-03 | 5,80E-02 | N | | | | | |
| SVEP1 | 2,98 | 8,00E-04 | 1,05E-02 | | | | A | | |
| ROBO1 | 2,95 | 5,89E-08 | 6,87E-06 | N | ASD | | A | M | P |
| ATP1B2 | 2,94 | 7,00E-04 | 9,90E-03 | | | ICN | A | M | |
| FLRT3 | 2,93 | 4,97E-05 | 1,30E-03 | N | ASD | | A | M | |
| DNER | 2,92 | 2,74E-02 | 1,38E-01 | N | ASD | | | M | |
| EDIL3 | 2,91 | 4,00E-04 | 6,60E-03 | | | | A | | |
| SLC1A1 | 2,91 | 2,28E-07 | 1,96E-05 | | | ICN | | | |
| FRMPD4 | 2,89 | 2,02E-02 | 1,11E-01 | | ASD | | | | |
| DTNA | 2,88 | 1,00E-04 | 2,50E-03 | N | | | | | |
| DLX1 | 2,87 | 3,98E-05 | 1,10E-03 | N | | | | | |
| MAPT | 2,86 | 4,96E-06 | 2,00E-04 | N | ASD | ICN | | M | |
| NEDD4L | 2,86 | 5,05E-07 | 3,51 E-05 | | ASD | ICN | | | |
| CACNA1 B | 2,85 | 6,83E-06 | 3,00E-04 | N | | ICN | | | |
| LAMA2 | 2,84 | 8,43E-07 | 5,36E-05 | N | ASD | | A | M | |
| CDKL2 | 2,83 | 1,00E-04 | 2,30E-03 | | | | | | P |
| NLGN3 | 2,82 | 5,30E-03 | 4,49E-02 | N | ASD | | A | | |
| JUN | 2,79 | 1.01E-02 | 6,97E-02 | N | ASD | | | M | P |
| L1CAM | 2,79 | 1,15E-02 | 7,60E-02 | N | ASD | | A | M | |
| CNTNAP3B | 2,76 | 3,00E-04 | 5,60E-03 | | | | A | | |
| ANK3 | 2,74 | 3,06E-07 | 2,41 E-05 | N | ASD | ICN | A | | |
| UST | 2,74 | 7,30E-03 | 5,58E-02 | | ASD | | | | |
| NEUROD2 | 2,73 | 2,90E-06 | 1,00E-04 | N | ASD | | | | |
| ANKS1 B | 2,72 | 9,10E-05 | 2,10E-03 | | ASD | | | | |
| SRGAP2 | 2,71 | 1,07E-05 | 4,00E-04 | N | | | A | M | P |
| DACT1 | 2,7 | 3,04E-05 | 9,00E-04 | | | | | | P |
| NRP1 | 2,7 | 6,90E-03 | 5,37E-02 | N | ASD | | | M | P |
| SEZ6L | 2,7 | 4,93E-05 | 1,30E-03 | | ASD | | | | |
| MADD | 2,67 | 2,00E-04 | 4,00E-03 | | | | | | P |
| ASTN1 | 2,65 | 3,00E-04 | 4,60E-03 | N | | | A | M | |
| DLG4 | 2,65 | 4,31E-07 | 3,11E-05 | N | ASD | | | | |
| SCN2A | 2,65 | 1,30E-03 | 1,53E-02 | N | | ICN | | | |
| ITPR1 | 2,64 | 8,00E-04 | 1,09E-02 | | | ICN | | | |
| PCDHB6 | 2,64 | 4,00E-04 | 7,10E-03 | N | ASD | | A | | |
| NPTX1 | 2,63 | 2,50E-03 | 2,58E-02 | N | ASD | | | | |
| GRIK2 | 2,62 | 2,00E-04 | 4,40E-03 | N | | | | | |
| TMEM63B | 2,62 | 2,00E-03 | 2,19E-02 | | | ICN | | | |
| DOCK9 | 2,61 | 5,78E-05 | 1,50E-03 | | | | A | | |
| NRXN2 | 2,61 | 3,00E-04 | 5,20E-03 | N | ASD | ICN | A | | |
| OPTN | 2,61 | 2,56E-06 | 1,00E-04 | | | | | | P |
| CDH8 | 2,6 | 1,88E-05 | 6.00E-04 | | | | A | | |
| PRKAR2B | 2,6 | 2,23E-09 | 6,28E-07 | | | | | | P |
| F3 | 2,59 | 1,40E-03 | 1,65E-02 | | | | | M | P |
| SCG2 | 2,59 | 1,49E-02 | 9,07E-02 | | | | | M | P |
| APC | 2,58 | 5,19E-08 | 6,16E-06 | | | | A | M | P |
| NCAM2 | 2,58 | 8,00E-04 | 1,14E-02 | N | ASD | | A | | |
| PPP3CA | 2,57 | 3,20E-07 | 2,51 E-05 | | ASD | ICN | | | P |
| TXN | 2,56 | 8,50E-03 | 6,23E-02 | | | | | | P |
| NUP93 | 2,55 | 2,25E-05 | 7,00E-04 | | | | | M | |
| ARHGAP18 | 2,54 | 1,04E-02 | 7,09E-02 | | | | A | | |
| CDH23 | 2,53 | 3,47E-02 | 1,60E-01 | | | ICN | A | | |
| CHRNB2 | 2,53 | 2,82E-05 | 8,00E-04 | N | ASD | ICN | | | P |
| PLPPR4 | 2,52 | 1,00E-04 | 2,40E-03 | | ASD | | | | |
| DPF1 | 2,51 | 1,01E-06 | 6,17E-05 | N | | | | | |
| VCAN | 2,51 | 8,48E-08 | 9,09E-06 | N | | | A | M | |
| ANK2 | 2,5 | 3,83E-05 | 1,10E-03 | | | ICN | | | |
| OMG | 2,5 | 9,40E-03 | 6,63E-02 | | ASD | | A | | |
| SPRY3 | 2,5 | 5,00E-03 | 4,29E-02 | | ASD | | | | |
| SPRY3 | 2,5 | 5,00E-03 | 4,29E-02 | | ASD | | | | |
| COL12A1 | 2,49 | 8,20E-03 | 6,05E-02 | | | | A | | |
| MAGI2 | 2,49 | 7.43E-06 | 3,00E-04 | N | | | | M | P |
| SMARCD3 | 2,49 | 1,00E-04 | 2,30E-03 | N | | | | | P |
| CD200 | 2,48 | 4,37E-06 | 2,00E-04 | | | | A | | |
| DAB2IP | 2,48 | 4,14E-05 | 1,10E-03 | N | ASD | | A | M | P |
| PTPRO | 2,48 | 2,00E-04 | 4,20E-03 | N | ASD | | A | | |
| RTN1 | 2,48 | 2,00E-04 | 3,50E-03 | N | | | | | |
| SCRT2 | 2,48 | 1,40E-03 | 1,61 E-02 | N | | | | M | |
| ETV5 | 2,47 | 1,00E-04 | 2,30E-03 | N | ASD | | | | P |
| SRGAP2C | 2,47 | 1,75E-06 | 9,39E-05 | N | | | | M | |
| ADAM22 | 2,46 | 5,07E-08 | 6,11E-06 | N | | | A | | |
| CABLES2 | 2,46 | 8,93E-05 | 2,00E-03 | | | | | | P |
| JAK2 | 2,46 | 1,99E-02 | 1,11E-01 | N | ASD | | A | M | P |
| PRKACB | 2,46 | 1,18E-06 | 6,98E-05 | | | | | | P |
| SULF2 | 2,46 | 7,10E-03 | 5,46E-02 | N | | | | | P |
| SYT14 | 2,46 | 3,60E-05 | 1,00E-03 | N | | ICN | | | |
| GLRA1 | 2,45 | 1,00E-03 | 1,30E-02 | N | | ICN | | | |
| HEY2 | 2,45 | 5,50E-03 | 4,58E-02 | | | | | | P |
| KLF7 | 2,45 | 9,08E-07 | 5,68E-05 | | ASD | | | | |
| NFIA | 2,45 | 7,15E-06 | 3,00E-04 | | ASD | | | | |
| CAMK2B | 2,44 | 1,00E-03 | 1,25E-02 | N | ASD | ICN | | | |
| SCN8A | 2,44 | 1,16E-05 | 4,00E-04 | N | | ICN | | | |
| SYP | 2,44 | 5,97E-08 | 6,92E-06 | N | | | | | |
| APLP1 | 2,43 | 4,36E-06 | 2,00E-04 | N | | | A | | |
| CLASP2 | 2,43 | 3,20E-03 | 3,04E-02 | | ASD | | A | M | |
| PBX3 | 2,43 | 1,54E-06 | 8,57E-05 | N | | | | | |
| ZIC5 | 2,43 | 5,10E-03 | 4,33E-02 | N | | | | | |
| DOCK4 | 2,42 | 2,00E-04 | 4,30E-03 | | | | | M | |
| JAK1 | 2,42 | 1,34E-06 | 7,80E-05 | | | | | M | P |
| SCN3A | 2,42 | 3,00E-04 | 5,70E-03 | N | | ICN | | | |
| SRGAP2B | 2,42 | 3,79E-07 | 2,85E-05 | N | | | | | |
| CADM3 | 2,41 | 5,00E-04 | 8,20E-03 | | | | A | | |
| CHD5 | 2,41 | 4,86E-05 | 1,30E-03 | N | | | | | P |
| CITED2 | 2,41 | 1,41 E-05 | 5.00E-04 | N | | | A | M | P |
| NAP1L2 | 2,41 | 2,00E-04 | 4,40E-03 | N | | | | | |
| CACNB2 | 2,4 | 1,00E-03 | 1,27E-02 | N | | ICN | | | |
| PCDH9 | 2,4 | 6,24E-06 | 3,00E-04 | | | | A | | |
| CACNG2 | 2,39 | 1,51E-05 | 5.00E-04 | N | | | | | |
| SLCO3A1 | 2,39 | 7,00E-04 | 1,01 E-02 | | | ICN | | | |
| ASIC1 | 2,38 | 7,30E-03 | 5,61E-02 | N | | ICN | | | |
| CNGA3 | 2,38 | 9,00E-04 | 1,15E-02 | | | ICN | | | |
| DUSP26 | 2,38 | 5,80E-03 | 4,75E-02 | | | | A | | |
| PLXNC1 | 2,38 | 6,39E-05 | 1,60E-03 | N | ASD | | A | M | |
| SPTBN1 | 2,38 | 1,37E-05 | 5,00E-04 | | ASD | | A | | |
| CEP112 | 2,36 | 8,44E-05 | 1,90E-03 | | ASD | | | | |
| MCOLN1 | 2,36 | 1,00E-04 | 2,40E-03 | | | ICN | | | |
| NRCAM | 2,36 | 1,06E-08 | 1,83E-06 | N | ASD | | A | M | |
| APBB1 | 2,35 | 3,09E-05 | 9,00E-04 | N | ASD | | | | P |
| ELK1 | 2,35 | 3,80E-03 | 3,48E-02 | N | | | | | |
| NRXN3 | 2,35 | 1,62E-02 | 9,60E-02 | N | ASD | ICN | A | | |
| SLC6A1 | 2,35 | 2,17E-05 | 7,00E-04 | N | | ICN | | | |
| VSTM2L | 2,35 | 3,00E-04 | 5,00E-03 | N | | | | | |
| BCAN | 2,34 | 8,40E-03 | 6,17E-02 | N | | | A | | |
| CAMSAP3 | 2,34 | 7,00E-04 | 9,60E-03 | N | | | A | | |
| KIRREL3 | 2,34 | 8,76E-06 | 3,00E-04 | N | ASD | | A | M | |
| SLITRK1 | 2,34 | 1,80E-03 | 2,03E-02 | | ASD | | | | |
| FZD1 | 2,33 | 1,00E-04 | 2,60E-03 | N | ASD | | | | |
| LDLRAD4 | 2,33 | 6.00E-04 | 9,40E-03 | | | | | M | |
| MITF | 2,33 | 1,40E-03 | 1,62E-02 | | | | | M | |
| PDZD2 | 2,33 | 7,10E-03 | 5,51 E-02 | | | | A | | |
| SYN1 | 2,33 | 3,30E-03 | 3,10E-02 | N | | ICN | | | |
| GDF9 | 2,32 | 4,77E-05 | 1,30E-03 | | | | | | P |
| PCDHB14 | 2,32 | 1,64E-06 | 9,07E-05 | | ASD | | A | | |
| KCNMA1 | 2,31 | 9,60E-03 | 6,74E-02 | | | ICN | | | |
| NCAM2 | 2,31 | 2,00E-04 | 4,20E-03 | N | ASD | | A | | |
| RORA | 2,31 | 6,20E-03 | 4,98E-02 | | | | | | P |
| EPHA7 | 2,3 | 3,78E-07 | 2,85E-05 | N | ASD | | A | | |
| RAB11 B | 2,3 | 3,30E-03 | 3,13E-02 | | | ICN | A | | |
| DOK4 | 2,29 | 3,80E-03 | 3,48E-02 | N | | | | | |
| OPHN1 | 2,29 | 5.00E-04 | 7,10E-03 | N | ASD | | | M | |
| RERE | 2,29 | 4,00E-04 | 6,30E-03 | | ASD | | | M | P |
| RIMS2 | 2,29 | 7,00E-04 | 9,40E-03 | N | ASD | ICN | | | |
| KCTD13 | 2,28 | 2,50E-03 | 2,56E-02 | | | | | M | |
| RBFOX2 | 2,28 | 4,67E-08 | 5,72E-06 | N | ASD | | | M | P |
| ELAVL3 | 2,27 | 3,05E-05 | 9,00E-04 | N | | | | | |
| KDM4C | 2,27 | 7,22E-05 | 1,70E-03 | | | | | | P |
| STX1B | 2,27 | 8,00E-04 | 1,07E-02 | N | | ICN | | | |
| VASH1 | 2,27 | 1,21 E-05 | 4,00E-04 | | | | | M | P |
| BEX1 | 2,25 | 3,20E-03 | 3,04E-02 | N | | | | | |
| LPAR1 | 2,25 | 3,00E-04 | 5,00E-03 | N | | | | | |
| MMP24 | 2,25 | 1,01 E-02 | 6,95E-02 | N | | | A | | |
| MYO5A | 2,25 | 7,67E-06 | 3,00E-04 | | ASD | | | | |
| PCDHB4 | 2,25 | 3,68E-06 | 2,00E-04 | N | ASD | | A | | |
| ADRB2 | 2,24 | 2,60E-03 | 2,61E-02 | | | ICN | | | |
| ALK | 2,23 | 1,70E-03 | 1,90E-02 | N | | | | | P |
| CLASP2 | 2,23 | 6,89E-05 | 1,70E-03 | | ASD | | A | M | |
| GNAO1 | 2,23 | 6,69E-06 | 3,00E-04 | N | | ICN | | | |
| CACNA2D2 | 2,21 | 2,32E-05 | 7,00E-04 | N | | | | | |
| GRIK1 | 2,21 | 6,70E-03 | 5,30E-02 | N | | | | | |
| PPP2R2B | 2,21 | 2,80E-03 | 2,75E-02 | | | | | | P |
| NQO2 | 2,2 | 9,62E-06 | 4,00E-04 | N | | | | | P |
| RASSF5 | 2,2 | 3,09E-05 | 9,00E-04 | | | | | | P |
| FAT3 | 2,19 | 1,00E-04 | 2,70E-03 | | | | A | | |
| CDHR3 | 2,18 | 1,20E-03 | 1,45E-02 | | | | A | | |
| DGKI | 2,18 | 1,00E-04 | 2,30E-03 | N | | ICN | | | |
| DPYSL3 | 2,18 | 2,29E-05 | 7,00E-04 | N | ASD | | | M | |
| NEUROD1 | 2,18 | 3,46E-02 | 1,60E-01 | N | | | | | P |
| PCDHB5 | 2,18 | 3,00E-04 | 5,20E-03 | | ASD | | A | | |
| PIK3CB | 2,18 | 1,32E-05 | 5,00E-04 | | | | A | M | P |
| SLCO1C1 | 2,18 | 9,50E-03 | 6,71 E-02 | | | ICN | | | |
| ECEL1 | 2,17 | 5,00E-04 | 8,00E-03 | N | | | | | |
| IGSF11 | 2,17 | 2,40E-03 | 2,49E-02 | | | | A | | |
| ZFHX3 | 2,17 | 1,70E-03 | 1,87E-02 | N | | | | | P |
| CLASP2 | 2,16 | 4,20E-03 | 3,75E-02 | | ASD | | A | M | |
| ICA1 | 2,16 | 3,42E-05 | 1,00E-03 | N | | ICN | | | |
| PDZRN3 | 2,16 | 6.00E-04 | 8,40E-03 | N | | | | | |
| ADGRB2 | 2,15 | 3,00E-04 | 5,70E-03 | N | ASD | | | | |
| GABRB3 | 2,15 | 4,55E-05 | 1,20E-03 | N | | | | | |
| CYB5D2 | 2,14 | 3,90E-03 | 3,58E-02 | N | | | | | |
| HOMER1 | 2,14 | 6.00E-04 | 9,10E-03 | | | ICN | | | |
| ATP7B | 2,13 | 1,05E-05 | 4,00E-04 | | | ICN | | | |
| DCC | 2,13 | 2,51E-06 | 1,00E-04 | N | ASD | | | M | |
| DCHS1 | 2,13 | 9,40E-05 | 2,10E-03 | N | | | A | M | P |
| MMP16 | 2,13 | 1,00E-04 | 3,00E-03 | | | | | | P |
| PCDHB8 | 2,13 | 4,00E-04 | 6,90E-03 | | | | A | | |
| SLCO4C1 | 2,13 | 6,83E-05 | 1,70E-03 | | | ICN | | | |
| SLIT2 | 2,13 | 5,00E-04 | 7,90E-03 | N | ASD | | | M | |
| GLRA3 | 2,12 | 2,00E-04 | 3,90E-03 | N | | | | | |
| MDGA2 | 2,12 | 6,97E-05 | 1,70E-03 | N | | | | | |
| PCDHB13 | 2,12 | 3,48E-05 | 1,00E-03 | | ASD | | A | | |
| SEMA3F | 2,12 | 9,29E-07 | 5,76E-05 | N | ASD | | | M | |
| FAM83B | 2,11 | 2,80E-03 | 2,79E-02 | | | | | | P |
| PLXNA4 | 2,11 | 1,29E-02 | 8,23E-02 | N | ASD | | | M | |
| FCHSD2 | 2,1 | 4,00E-04 | 6,50E-03 | N | | | | | |
| KIF5A | 2,1 | 3,92E-05 | 1,10E-03 | | ASD | | | | |
| SEMA4A | 2,09 | 1,70E-03 | 1,87E-02 | | ASD | | | M | |
| ATAT1 | 2,08 | 6,67E-05 | 1,60E-03 | N | | | | | |
| MEIS1 | 2,08 | 1,30E-02 | 8,26E-02 | N | | | | | |
| NAIP | 2,08 | 5,00E-04 | 7,90E-03 | N | ASD | | | | |
| PCDHB10 | 2,08 | 5,21E-06 | 2,00E-04 | | ASD | | A | | |
| INPP5F | 2,07 | 2,42E-05 | 7,00E-04 | | ASD | | | | |
| MBD5 | 2,07 | 6,38E-05 | 1,60E-03 | N | | | | | |
| MLC1 | 2,07 | 7,00E-04 | 1,02E-02 | | | ICN | | | |
| SORBS1 | 2,07 | 1,50E-03 | 1,74E-02 | | | | A | | |
| USPL1 | 2,07 | 8,75E-05 | 2,00E-03 | | | | | | P |
| LRP8 | 2,06 | 9,32E-05 | 2,10E-03 | | ASD | | | | |
| NCAM1 | 2,06 | 5,00E-04 | 7,90E-03 | N | ASD | | A | | |
| PCDH15 | 2,06 | 8,46E-06 | 3,00E-04 | | | | A | | |
| SHISA7 | 2,06 | 7,58E-05 | 1,80E-03 | N | | | | | |
| HLA-DMB | 2,05 | 1,90E-03 | 2,06E-02 | | | | | | P |
| PRKCZ | 2,05 | 9,00E-04 | 1,24E-02 | N | | | A | M | P |
| RORA | 2,05 | 7,20E-03 | 5,52E-02 | | | | | | P |
| RUFY3 | 2,05 | 2,02E-05 | 6,00E-04 | | ASD | | | M | |
| SLCO1A2 | 2,05 | 3,66E-06 | 2,00E-04 | | | ICN | | | |
| AZI2 | 2,04 | 7,00E-04 | 1,03E-02 | | | | | | P |
| GPRIN1 | 2,04 | 2,00E-04 | 3,20E-03 | N | | | | | |
| MTSS1 | 2,04 | 1,30E-03 | 1,52E-02 | | | | A | | P |
| SORBS2 | 2,04 | 1,60E-03 | 1,83E-02 | | | | A | M | |
| TCF4 | 2,04 | 6,77E-06 | 3,00E-04 | N | | | | | |
| BMPR2 | 2,03 | 3,75E-06 | 2,00E-04 | | ASD | | | M | P |
| HHIP | 2,03 | 4,00E-03 | 3,61E-02 | N | | | | | P |
| SPAST | 2,03 | 6,44E-07 | 4,30E-05 | | ASD | | | | |
| ST8SIA2 | 2,03 | 8,00E-04 | 1,10E-02 | N | | | | | |
| PLK2 | 2,02 | 2,35E-02 | 1,23E-01 | | | | | | P |
| WNT2B | 2,02 | 3,10E-03 | 3,01 E-02 | N | | | | | P |
| ZNF280D | 2,02 | 4,33E-06 | 2,00E-04 | | ASD | | | | |
| LZTS1 | 2,01 | 5,78E-05 | 1,50E-03 | | ASD | | | | P |
| NTM | 2,01 | 1,63E-02 | 9,62E-02 | N | | | A | | |
| PRKCA | 2,01 | 2,20E-03 | 2,36E-02 | | | | A | M | P |
| SYT16 | 2,01 | 6,87E-05 | 1,70E-03 | N | | ICN | | | |
| VAMP2 | 2,01 | 8,00E-04 | 1,13E-02 | N | | ICN | | | |

**Table 1: List of genes up-regulated in ND7 GCNF-VP16 and filtered for their associated function.** Gene ontology annotation for biological processes was performed by DAVID Bioinformatic Resources 6.8. Gene ontology annotation was then filtered for GO terms including (1) "nervous system" OR "neuro", (2) "axon" OR "synapse" OR "dendrite", (3) "ion transport" OR "channel" or "neurotransmitter" (NT), (4) "adhesion", (5) "migration", (6) "cell cycle" OR "proliferation". The number of genes found for each inquiry is indicated in italics. The function categories assigned for each gene are indicated by the respective abbreviations. Gene expression fold change of GCNF-VP 16 ND7 samples (n = 3) compared to Eto and ctr ND7 samples (n = 6). P-values and FDR P-values are indicated.

In preferred embodiments, the transcription factor comprises or consists of a polypeptide having the amino acid sequence according to SEQ ID NO: 8 or exhibiting at least 85%, at least 90%, at least 95% sequence identity to SEQ ID NO: 8 and having a transactivation activity of at least 2-fold, in particular at least 10-fold induction of the expression of a gene selected from the group consisting of ZIC3, NFIB, BCL11A, NEUROD1, and NEUROG2.

In preferred embodiments, the transcription factor comprises or consists of a polypeptide having the amino acid sequence according to SEQ ID NO: 8 or exhibiting at least 85%, at least 90%, at least 95%, at least 97% or at least 99% sequence identity to SEQ ID NO: 8 and having a transactivation activity of at least 10-fold, particularly at least 25-fold, more particularly at least 40-fold induction of the expression of BCL11A. BCL11A is a transcription factor involved in cell migration and differentiation in the neural lineage (Simon et al., 2020). Herein, the inventors provide experimental evidence suggesting that BCL11A is a direct target gene of GCNF and induced by GCNF-VP16.

In preferred embodiments, the transcription factor comprises or consists of a polypeptide having the amino acid sequence according to SEQ ID NO: 8 or exhibiting at least 85%, at least 90%, at least 95%, at least 97% or at least 99% sequence identity to SEQ ID NO: 8 and having a transactivation activity of at least 2-fold induction of the expression of NEUROD1 and/or NEUROG1.

In preferred embodiments, the transcription factor comprises or consists of a polypeptide having the amino acid sequence according to SEQ ID NO: 8 or exhibiting at least 85%, at least 90%, at least 95%, at least 97% or at least 99% sequence identity to SEQ ID NO: 8 and having a transactivation activity of at least 50-fold, particularly at least 100-fold, more particularly at least 200-fold induction of the expression of ZIC3.

In especially preferred embodiments, the transcription factor comprises or consists of a polypeptide having the amino acid sequence according to SEQ ID NO: 31 or exhibiting at least 85%, at least 90%, at least 95%, at least 97% or at least 99% sequence identity to SEQ ID NO: 31 and having a transactivation activity as described above in the embodiments relating to SEQ ID NO: 8.

In a second aspect, the present invention relates to a method of converting one or more NCs into one or more induced neuronal cells, comprising (a) providing NCs; and (b) inhibiting the expression of GCNF in the NCs; thereby producing induced neuronal cells. Inhibition of GCNF expression can be achieved by various techniques known in the art, e.g. by RNAi techniques, knock-out techniques or using the CRISPR/Cas9 technology.

In a third aspect, the present invention relates to a method of converting one or more NCs into one or more induced neuronal cells, comprising (a) providing NCs; and (b) expressing a GCNF target gene in the NCs or contacting the NCs with the gene product of a GCNF target gene; thereby producing induced neuronal cells. It can be envisioned that the expression of the GCNF target gene is induced by inhibition of GCNF expression or expression of a dominant-negative GCNF, or expression of the TF of the first aspect of the invention. Inhibition of GCNF expression can be achieved by various techniques known in the art, e.g. by RNAi techniques, knock-out techniques or using the CRISPR/Cas9 technology. Expression of GCNF target gene can be also induced by using a multiplexed CRISPR activation method, which involves usage of an activator-domain fusion based dCas9 transcription activator in combination with multiple guideRNAs directed to the target genes (reviewed by McCarty et al., 2020). Alternatively, expression of GCNF target genes may be achieved by expressing/or delivering a dCas9 transcription activator together with a guideRNA designed to target the DR0 response element (Borys et al., 2020) (see SEQ ID NO 23). In preferred embodiments of the third aspect, the GCNF target gene is selected from the group consisting of ZIC3, NFIB, BCL11A, NEUROD1, and NEUROG2. In even more preferred embodiments of the third aspect, the GCNF target gene is selected from the group consisting of BCL11A, NEUROD1, and NEUROG2. In most preferred embodiments of the third aspect, the GCNF target gene is BCL11A.

In preferred embodiments of the first, second or third aspect of the invention, the induced neuronal cell is characterized by one or more of the following features in comparison to the NC:
a. increased expression of a neuronal marker selected from the group consisting of DCX, βIII-Tubulin (TUBB3), MAP2, Synaptophysin (SYP), PSD95 (DLG4) and CD200;
b. decreased expression of an undifferentiated NC marker selected from the group consisting of Nestin (NES), SOX1, SOX2, PAX2, PAX6, DACH1, PLZF, HES1, GPM6A, RFX4, POU3F3 and CD49f (ITGA6); in particular selected from the group consisting of NES, SOX1, SOX2, PAX2, PAX6 and ITGA6;
c. decreased expression of proliferation markers selected from the group consisting of MKI67, PCNA and CylinD1 (CCND1), CyclinE1 (CCNE1);
d. increased expression of genes of gene ontology classification GO: 0010975∼regulation of neuron projection development, GO:0061564∼axon development, GO:0050808∼synapse organization, GO:0099177∼trans-synaptic signalling, GO:0042391∼regulation of membrane potential, GO:0030900∼forebrain development, GO:0098742∼cell-cell adhesion via plasma-membrane adhesion molecules;
e. decreased expression of genes of gene ontology classification G0:0061351∼neural precursor cell proliferation, GO:0044772∼mitotic cell cycle phase transition, GO:0043062∼extracellular structure organization, GO:0002009∼morphogenesis of an epithelium;
f. increased formation of neurite processes;
g. increased neurite length; and/or
h. increased calcium influx in response to membrane depolarization.

In comparison to an induced neuronal cell that was generated by other methods known in the art, the induced neuronal cell generated by the method according to the first aspect of the invention is characterized by one or more of features a-f.

The skilled person is aware that the induced neuronal cell generated by the method according to the invention is usually present within a culture of cells comprising or consisting of a plurality of substantially identical or very similar induced neuronal cells (induced neuronal cell culture). In preferred embodiments of the first or second aspect of the invention, an induced neuronal cell culture comprising or consisting of a plurality of said induced neuronal cells is characterized by reduced cell cluster formation, in particular wherein the size of clusters is reduced and/or the number of clusters with a size of above 4000 µm² is reduced by at least 50%, at least 60%, at least 70%, at least 75%, or at least 80%. The reduced cluster formation is observed in comparison to cell cultures comprising induced neuronal cells generated by other methods known in the art.

In preferred embodiments of the first, second or third aspect of the invention, an induced neuronal cell culture comprising or consisting of a plurality of said induced neuronal cells is characterized by reduced cell density within the cell clusters formed, in particular wherein the number of clusters with more than 30 cells is reduced by 3-fold compared to cell cultures comprising induced neuronal cells generated by other methods known in the art.

In preferred embodiments of the first, second or third aspect of the invention, early neuronal differentiation is apparent within 48 hours, particularly 36 hours, more particularly 24 hours after contacting the NC with the TF. In preferred embodiments, early neuronal differentiation at 24 hours is characterized by the formation of neurite processes. In preferred embodiments, early neuronal differentiation at 48 hours is characterized by an increased formation of neurite processes.

In a fourth aspect, the present invention relates to a TF comprising a DNA binding moiety and a heterologous TAD, wherein the DNA binding moiety specifically binds to a direct repeat with zero spacing (DRO) response element.

Any embodiments of the TF described with respect to the method of the first aspect of the invention also apply to the fourth aspect of the invention. In preferred embodiments of the fourth aspect of the invention, the DNA binding moiety is a DBD. In more preferred embodiments, the DBD comprises or consists of a DBD of a TF selected from SF1/NR5A1, LRH-1/NR5A2 and Germ Cell Nuclear Factor (GCNF/NR6A1), preferably human SF1/NR5A1, LRH-1/NR5A2 or GCNF/NR6A1. In even more preferred embodiments, the DBD comprises or consists of a DBD of GCNF, preferably human GCNF. In even more preferred embodiments of the fourth aspect of the invention, the DBD comprises or consists of SEQ ID NO: 2. In most preferred embodiments, the DBD comprises or consists of SEQ ID NO: 3 or SEQ ID NO: 4.

In preferred embodiments of the fourth aspect of the invention, the TAD induces unfolding of chromatin structure, recruitment of a transcription initiation complex, initiation of transcription and/or elongation of the RNA transcript.

In preferred embodiments of the fourth aspect of the invention, the TAD comprises or consists of a TAD selected from the group consisting of the activation domain of a nuclear receptor, in particular of a steroid, thyroid or retinoid acid receptor; a synthetic or chimeric activation domain; a basic or acidic amino acid activation domain; a viral activation domain; an activation domain of the nine-amino-acid TAD (9aaTAD) family, in particular a 9aaTAD as comprised in Gal4, Oaf1, Pip2, Pdr1, Pdr3, Leu3, Teal, Pho4, Gln3, Gcn4, Msn2, Msn4, Rtg3, CREB, CREBaB6, E2A, MLL, p53-TADI, p53-TADII, FOXO3, NF-kB, NFAT, CEBPA/E, ESX, ELF3, ETV1, KLF2/4, EBNA2, VP16, HSF1, HSF2, HsfA, Gli3, Sox18, PIF, Dreb2a, MTF1, OREB1, WRKY45, NS1, MKL1, VP16, EBNA2, KBP220, ECapLL, P201, AH, or B42; a glutamine-rich activation domain, in particular as present in POU2F1 (Oct1), POU2F2 (Oct2) and Sp1); a proline-rich activation domain, in particular as present in c-jun, AP2 or Oct2; an isoleucine-rich activation domain, in particular as present in NTF-1; a Tat activation domain; a BP64 activation domain; a TAF-1 activation domain; a TAF-2 activation domain; a TAU-1 activation domain; a TAU-2 activation domain; a YAP activation domain, a bHLH protein activation domain, in particular as present in MYOD, ASCL1 or NGN2, or a fragment of said activation domains or a modified version thereof. In preferred embodiments, the TAD comprises or consists of the TAD of VP16, VP64, YAP or MYOD. In more preferred embodiments, the TAD comprises or consists of the TAD of VP16 or VP64.

In preferred embodiments of the fourth aspect of the invention, the TAD comprises or consists of SEQ ID NO: 5. In even more preferred embodiments, the TAD comprises or consists of SEQ ID NO: 6 or SEQ ID NO: 7.

One example of a TF according to the fourth aspect of the invention is a GCNF-VP16 fusion polypeptide comprising or consisting of the amino acid sequence of SEQ ID NO: 31. Another example of a TF according to the fourth aspect of the invention is a GCNF-VP16 fusion polypeptide comprising or consisting of the amino acid sequence of SEQ ID NO: 8, which corresponds to the polypeptide according to SEQ ID NO: 31, but wherein the last 10 amino acids are absent. The nucleotide and peptide sequences for GCNF and VP16 have been previously disclosed (GCNF GenBank accession number AF004291.1, VP16 GenBank accession number M60050.1) and coding regions for these genes may be amplified and/or expressed using techniques disclosed herein or as would be known to those of ordinary skill in the art. In certain embodiments, the TF comprises multiple domains, including polypeptides, or peptides or peptidomimetic domains. The TF may be isolated and purified in accordance with conventional methods of recombinant synthesis.

In preferred embodiments of the fourth aspect of the invention, the TF further comprises (a) an affinity tag to facilitate purification or identification of the protein; and/or (b) a cellular uptake signal that enhances transport of a polypeptide across a cellular membrane; and optionally (c) an additional nuclear localization sequence to enhance translocalization to the nucleus.

In a fifth aspect, the present invention relates to a nucleic acid encoding the TF of the fourth aspect of the invention. In certain embodiments, the nucleic acid is a DNA or RNA molecule. In certain embodiments, the nucleic acid further comprises an inducible promoter.

In a sixth aspect, the present invention relates to a vector comprising the nucleic acid of the fifth aspect of the invention. In preferred embodiments, the vector is selected from the group consisting of a lentiviral vector, an adenoviral vector, an adenovirus associated viral (AAV) vector) and a sendai virus vector.

In a seventh aspect, the present invention relates to a cell comprising the TF of the fourth aspect, the nucleic acid of the fifth aspect and/or the vector of the sixth aspect of the invention.

In preferred embodiments of the seventh aspect of the invention, the nucleic acid of the fifth aspect of the invention is comprised episomally in the cell or is integrated into in the genome of the cell, in particular in a safe harbor locus.

In preferred embodiments of the seventh aspect of the invention, the cell is a NC. In preferred embodiments, the NC is a neural stem cell (NSC) or neural progenitor cell (NPC), preferably human NSC or human NPC. In preferred embodiments of the sixth aspect of the invention, the cell is selected from the group of cells described as suitable host cells with regard to the method according to the first aspect of the invention. Such cells can be used for rapid production of neuronal cells. It is particularly useful when in such cells, the TF of the third aspect of the invention is under the control of an inducible promoter, e.g. a doxycycline-inducible promoter. The described cells may be conserved by cryo-banking. When needed, the cells may be thawed and expression of the TF may be induced, leading to differentiation of the cells into neuronal cells.

In further embodiments of the seventh aspect of the invention, the cell is an induced neuronal cell, i.e. a cell in which neuronal differentiation has already occurred due to the introduction of the TF according to the fourth aspect of the invention.

In an eighth aspect, the present invention relates to a cell produced by the method of the first, second or third aspect of the invention.

In a ninth aspect, the present invention relates to a microplate comprising the cell of the seventh or eighth aspect of the invention. In preferred embodiments, the microplate is suitable for high-content imaging (HCI) based assays. In preferred embodiments, neuronal induction of the cell of the seventh or eighth aspect of the invention is effected directly on the microplate. In the context of the present specification, the expression "microplate" relates to a multi-well tissue culture plate that is suitable for microscopic imaging of cells comprised in said wells.

In a tenth aspect, the present invention relates to the use of a cell of the seventh or eighth aspect of the invention as a disease model.

In an eleventh aspect, the present invention relates to the use of a cell of the seventh or eighth aspect of the invention in a method of drug screening.

In a twelfth aspect, the present invention relates to the cell of the seventh or eighth aspect of the invention for use in a method of treatment or prevention of a neurological condition, in particular neuronal damage, neuronal loss or a neurodegenerative disease. In certain embodiments, the method comprises cell transplantation therapy in the CNS. In some embodiments, the cell transplantation therapy is neuron transplantation therapy. In some such embodiments, the subject is contacted with a composition of induced neuronal cells according to the seventh or eighth aspect of the invention. In certain embodiments, these cells are generated from NCs that are derived from somatic cells derived from the subject. In some embodiments, the subject has a CNS condition. The cells are administered in a manner that permits them to graft to the intended tissue site and reconstitute or regenerate the functionally deficient area. In some embodiments, the CNS condition is a neurodegenerative disease, a neuropsychiatric disorder, a channelopathy, a lysosomal storage disorder, an autoimmune disease of the CNS, a cerebral infarction, a stroke, or a spinal cord injury.

The induced neuronal cells produced by the above *in vitro* methods may be used as a basic research or drug discovery tool, for example to evaluate the phenotype of a disease, to better understand the etiology of the disease, to identify target proteins for therapeutic treatment, to identify candidate agents with disease-modifying activity. For example, a candidate agent may be added to a cell culture comprising the induce neuronal cells, and the effect of the candidate agent assessed by monitoring output parameters such as cell survival, the ability of the cells to become depolarized, the extent to which the cells form synapses, and the like, by methods described herein and in the art. Parameters are quantifiable components of cells, particularly components that can be accurately measured, desirably using HTS campaigns. A parameter can be any cell component or cell product including cell surface determinant, receptor, protein or conformational or posttranslational modification thereof, lipid, carbohydrate, organic or inorganic molecule, nucleic acid, e.g. mRNA, DNA, etc. or a portion derived from such a cell component or combinations thereof. While most parameters will provide a quantitative readout, in some instances a semiquantitative or qualitative result will be acceptable. Readouts may include a single determined value, or may include mean, median value or the variance, etc. Characteristically a range of parameter readout values will be obtained for each parameter from a multiplicity of the same assays. Variability is expected and a range of values for each of the set of test parameters will be obtained using standard statistical methods with a common statistical method used to provide single values. Candidate agents of interest for screening include known and unknown compounds that encompass numerous chemical classes, primarily organic molecules, which may include organometallic molecules, inorganic molecules, genetic sequences, etc. An important aspect of the invention is to evaluate candidate drugs, including toxicity testing; and the like. Candidate agents include organic molecules comprising functional groups necessary for structural interactions, particularly hydrogen bonding, and typically include at least an amine, carbonyl, hydroxyl or carboxyl group, frequently at least two of the functional chemical groups. The candidate agents often comprise cyclic carbon or heterocyclic structures and/or aromatic or polyaromatic structures substituted with one or more of the above functional groups. Candidate agents are also found among biomolecules, including peptides, polynucleotides, saccharides, fatty acids, steroids, purines, pyrimidines, derivatives, structural analogs or combinations thereof. Included are pharmacologically active drugs, genetically active molecules, etc. Compounds of interest include chemotherapeutic agents, hormones or hormone antagonists, etc. Exemplary pharmaceutical agents suitable for screening are those described in, "The Pharmacological Basis of Therapeutics. Goodman and Gilman, McGraw-Hill, New York, N.Y., (1996), Ninth edition.

### FIGURE LEGENDS

**Fig. 1** shows consensus binding sites for GCNF according to TRANSFAC^{®} Release 2018.2. Position weight matrix of GCNF (V$GCNF_01) (SEQ ID NO: 34 - N₁N₂N₃N₄N₅N₆N₇TCAN₈N₉N₁₀N₁₁N₁₂N₁₃N₁₄N₁₅, wherein N₁ is selected from A, T, G and C, N₂ is selected from A, T, G and C, and preferably is T, N₃ is selected from A, T, G and C, and preferably is C, N₄ is selected from A, T, G and C, and preferably is A, N₅ is selected from A, T, G and C, and preferably is A, N₆ is selected from A, T, G and C, and preferably is G, N₇ is selected from A, T, G and C, and preferably is T or G, more preferably is T, N₈ is selected from A, T, G and C, and preferably is A, N₉ is selected from A, T, G and C, and preferably is G or T, more preferably is G, N₁₀ is selected from A, T, G and C, and preferably is T or G, N₁₁ is selected from A, T, G and C, and preferably is T, N₁₂ is selected from A, T, G and C, and preferably is C, N₁₃ is selected from A, T, G and C, and preferably is A, N₁₄ is selected from A, T, G and C and N₁₅ is selected from A, T, G and C). Occurrence frequencies of the 4 nucleotides are indicated by variable letter sizes.
**Fig. 2** shows that GCNF-VP16 accelerates neuronal differentiation of hPSC-derived smNPCs. To modulate GCNF activity, smNPCs were transduced with the indicated lentiviral transgenes and differentiated for 7 days in the presence of doxycycline to induce transgene expression. As benchmark, smNPCs carrying a NGN2 overexpression construct were used. (A) Cells transduced with GCNF, GCNF mutDBD, or GCNF-VP16 show increased GCNF protein levels, while GCNF protein levels were drastically reduced upon expression of GCNF shRNA. (B) smNPCs were stained with SOX2 (neural progenitor marker). Corresponding quantification of SOX2 positive cells indicates a reduction of neural progenitor cells upon GCNF-VP 16 and NGN2 expression. (C) Flow cytometry-based EDU incorporation assay shows an increased proliferation rate in GCNF cultures and a decreased proliferation rate in GCNF shRNA, GCNF-VP16 and NGN2 cultures. (D) Flow cytometry analysis for CD200 (indicating differentiated cells) and CD49f (indicating undifferentiated cells) and (E) staining with MAP2 (neuronal marker) demonstrate that overexpression of GCNF represses neuronal differentiation while GCNF shRNA, GCNF-VP16 or NGN2 promote neuronal differentiation. Data are presented as mean + SEM; n ≥ 3. Unpaired t-test; *, p ≤ 0.05; **, p ≤ 0.01; ***, p ≤ 0.001, compared to ctr. Scale bar = 100 µm.

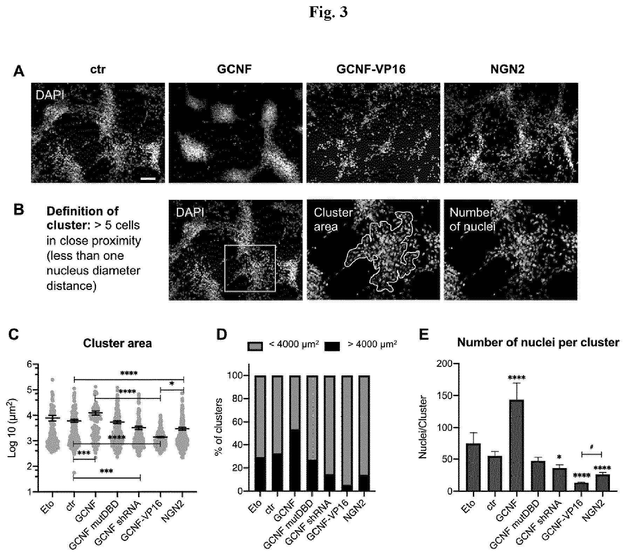

**Fig. 3** shows that GCNF and GCNF-VP16 affect cell cluster formation of differentiating smNPCs. **(A)** Nuclear DAPI staining of transgenic smNPCs cultures differentiated for 7 days reveals gross alterations in cell cluster formation in GCNF and GCNF-VP16 cultures compared to control. **(B)** A "cell cluster" was defined as a group of 5 or more cells in close proximity to each other. The area **(C, D)** and the number of nuclei per cluster **(E)** was determined. This analysis reveals that the cluster area and the cell density per cluster is increased in GCNF and decreased in GCNF-VP16 compared to controls. Scatter blot (C) and histogram (D, E) show data collected from three independent replicates; mean + SEM. Mann-Whitney test; *, #, p ≤ 0.05; ***, p ≤ 0.001; ****, p ≤ 0.0001, compared to ctr or the conditions indicated. Scale bar = 100 µm.

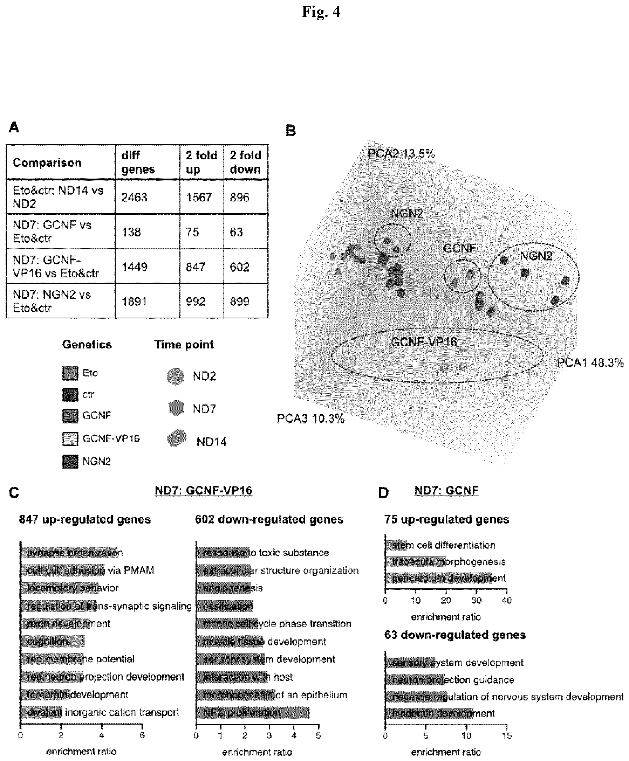

**Fig. 4** shows that GCNF-VP16 induces a neurogenic gene expression program in smNPCs. Microarray was used to assess transcript expression of smNPCs expressing the indicated transgenes at 2 (ND2), 7 (ND7) and 14 (ND14) days of differentiation. **(A)** Table summarizing results of the differential gene expression analysis for the indicated comparisons. **(B)** Principal component analysis (PCA) indicates different gene expression trajectories in GCNF, GCNF-VP16 and NGN2 samples compared to controls. **(C, D)** Gene ontology enrichment analysis for differentially expressed genes in GCNF-VP16 **(C)** and GCNF **(D)** ND7 samples. Abbreviations: NPC, neural precursor cell; PMAM, plasma-membrane adhesion molecules; reg, regulation of.

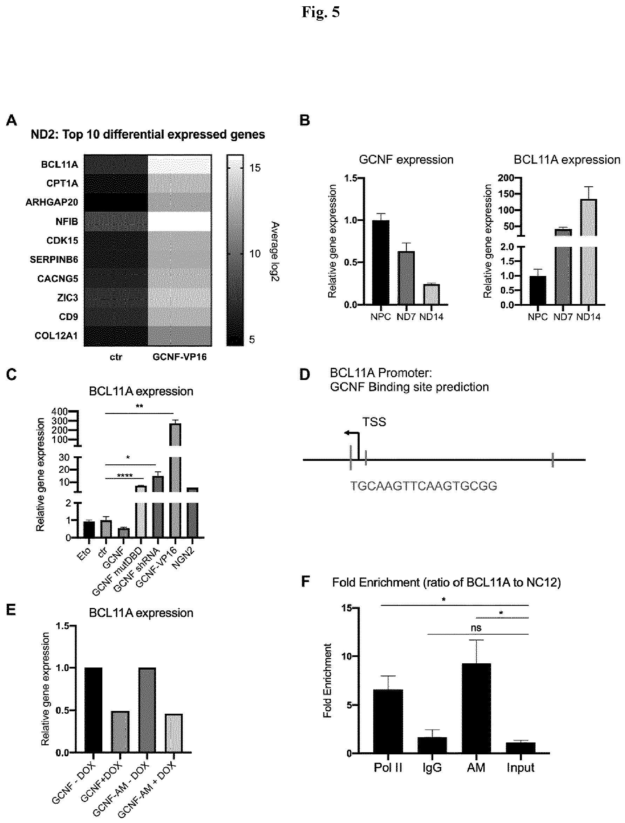

**Fig. 5** shows that GCNF and GCNF-VP16 regulates the expression of neuronal migration factor BCL11A by direct binding to its promoter. **(A)** Microarray expression data for the top 10 up-regulated genes in GCNF-VP16 smNPCs after 48 hours of transgene induction. **(B)** GCNF and BCL11A show opposite expression pattern during smNPC differentiation as assessed by qRT-PCR. (C) qRT-PCR analysis of BCL11A in transgenic smNPCs shows that BCL11A expression is regulated by GCNF modulation. While BCL11A expression is reduced upon GCNF overexpression, it is induced by expression of GCNF-VP 16 or GCNF shRNA. Interestingly, overexpression of NGN2 has a smaller effect on BCL11A expression than GCNF-VP16. Data are presented as mean + SEM, n = 3 (except NGN2 n = 1). Unpaired t-test; *, p ≤ 0.05; **, p ≤ 0.01; ****, p ≤ 0.0001. **(D)** Using TRANSFAC Match Tool, three binding sites for GCNF in the BCL11A promoter were identified. **(E-F)** To enable GCNF-ChIP, smNPCs were transduced with a lentiviral doxycycline-inducible construct expressing AM-tagged GCNF. **(E)** qRT-PCR for BCL11A confirms functionality of the GCNF-AM construct in smNPCs. **(F)** GCNF-AM smNPCs were treated for 2 days with doxycycline and subjected to the ChIP assay using the AM antibody. RNA-Polymerase II-precipitate was used as positive control, IgG- precipitate and pre-ChIP Input sample as negative control. ChIP-qPCR confirms enrichment of GCNF-AM-bound complexes on the BCL11A promoter fragment containing two predicted GCNF binding sites. As negative control, a ChIP-qPCR assay for a gene dessert element on chromosome 12 (NC12), was used. Fold-enrichment for BCL11A promoter fragment over NC12 levels were comparable for both GCNF-AM- and RNA-Polymerase II-precipitate. Data are represented as mean + SEM; n = 3. Unpaired t-test; *, p ≤ 0.05.

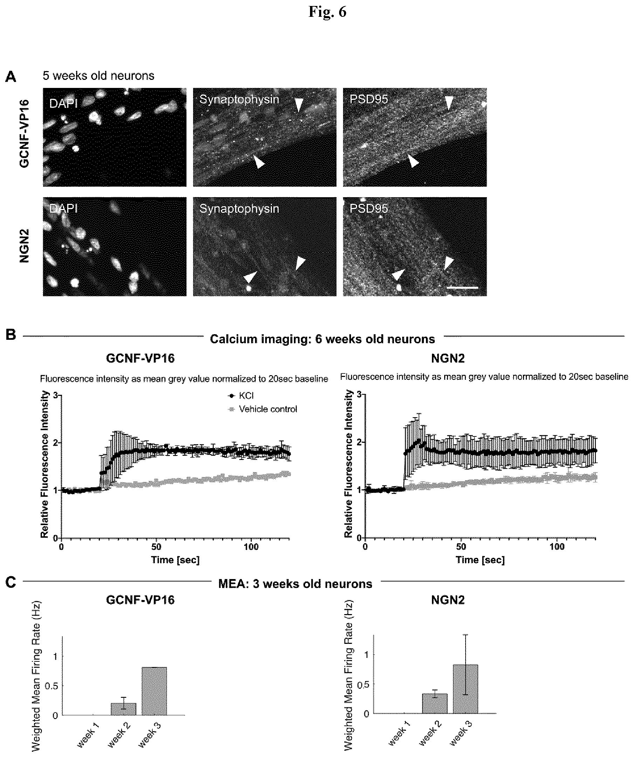

**Fig. 6** shows data indicating that GCNF-VP16 neurons and NGN2 neurons display a similar maturation status. smNPCs were transduced with GCNF-VP16 lentivirus or NGN2 lentivirus and differentiated for 1 to 2 weeks in the presence of doxycycline to induce transgene expression with following differentiation for 3 to 4 weeks in the presence of BDNF. **(A)** Staining for synaptic markers Synaptophysin and PSD95 indicate the presence of pre- and postsynaptic structure in the cultures. Arrow heads: Neuronal fiber, which is positive for Synaptophysin and PSD95, scale bar = 25 µm. **(B)** Ca²⁺ imaging of 6-week-old NGN2 and GCNF-VP 16 neurons demonstrate that both neuronal lines react to depolarization of their membrane potential with Ca²⁺ influx. **(C)** Analysis of the spontaneous activity of NGN2 or GCNF-VP16 induced neurons with multielectrode array (MEA) after 3 weeks of differentiation. The firing rate of spontaneous active cells normalized to the number of active electrodes (weighted mean firing rate) is the same for both cell lines and increases from week 2 of differentiation to week 3.
**Fig. 7** shows that the effect of GCNF-VP16 as inducer of neuronal differentiation is already apparent within 48 hours of transgene induction. smNPCs expressing the indicated transgenes were treated for 48 hours with differentiation medium and doxycycline to induce transgene expression. **(A, B)** Staining for the neuronal marker MAP2 and quantification of MAP2 positive cells demonstrates that GCNF-VP16 and NGN2 promotes neuronal differentiation. **(C)** The majority of the cells still stain positive for neural progenitor marker SOX2. **(D)** Quantification of cells stained positive for the proliferation marker KI67 and **(E)** flow-cytometry based EDU incorporation assay show a reduced proliferation rate in GCNF-VP16 and NGN2 cultures. Data are presented as mean + SEM; n = 3. Unpaired t test; *, p ≤ 0.05; **, p ≤ 0.01. Scale bar = 50 µm.

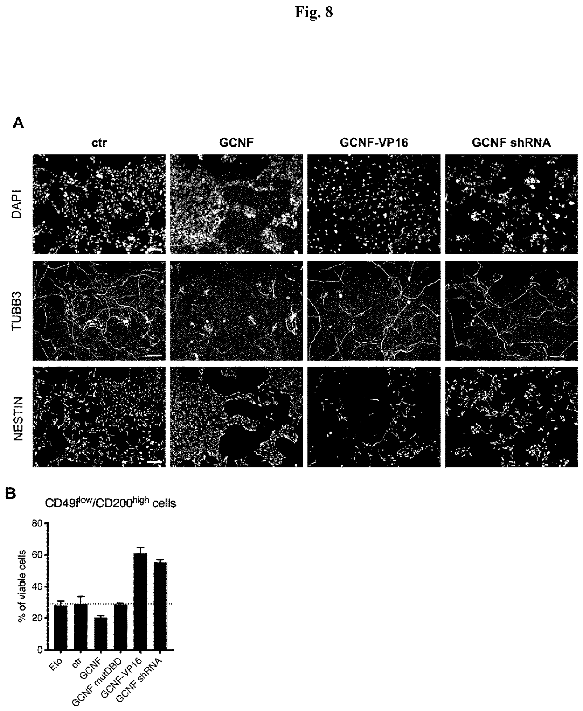

**Fig. 8** shows that GCNF-VP16 accelerates neuronal differentiation of hPSC-derived lt-NES cells. (A) Lt-NES cells were transduced with the indicated lentiviral constructs and differentiated for 7 days in the presence of doxycycline to induce transgene expression. Staining with NESTIN (marker for undifferentiated NSCs) and β-III Tubulin (neuronal marker) demonstrates that overexpression of GCNF represses neuronal differentiation while expression of GCNF-VP16 or GCNF shRNA promotes neuronal differentiation. **(B)** Flow cytometry-based quantification for CD200 (associated with differentiated neuronal cells) and CD49f (associated with undifferentiated NPCs) confirms the increase in neuronal differentiation induced by either GCNF-VP16 or GCNF shRNA. Treatment with Notch inhibitor DAPT as known neurogenic substance (Borghese et al., 2010) was used as a positive control.

### EXAMPLES

This invention describes the combination of human GCNF, which acts as a repressor of target gene expression (Cooney et al., 1998), with the VP16 transcription activation domain (TAD). Surprisingly, the inventors have found that the expression of GCNF-VP16 as GCNF-transactivator in NPCs results in accelerated neuronal differentiation of these cells in single cell resolution.

### Methods

### Cell culture

Small molecule NPCs (smNPCs) were generated from iLB-C-31F-rl hiPSCs. Experiments were performed between passage 14 and passage 26. To induce neuronal differentiation, smNPCs were re-plated on Geltrex-coated dishes and cultured in differentiation medium consisting of DMEM-F12 (Invitrogen)/Neurobasal (Invitrogen) 50:50, 1:200 N2 supplement (Invitrogen), 1:100 B27 supplement with vitamin A (Invitrogen), 1% penicillin/streptomycin (Invitrogen) and 1% glutamine (Invitrogen)). Transgene expression was induced by adding 4 µg/ml doxycycline to the differentiation medium.

Human lt-NES cells were generated as described in Roese-Koerner et al., 2016 from I3 hES cells. I3 lt-NES cells were maintained on PO/LN- or Geltrex-coated TC dishes with EGF and FGF2 (BD Biosciences) as previously described in Koch et al., 2009. Differentiation was performed on Geltrex-coated TC dishes in differentiation medium devoid of growth factors.

For pharmacological inhibition of the Notch signaling pathway, DAPT (N-[N-(3,5-difluorophenacetyl)-L-alanyl]-S-phenylglycine t-butyl ester, Sigma-Aldrich) was dissolved in DMSO (Sigma-Aldrich) and used at a final concentration of 5 mM.

### Cloning of constructs and generation of lentiviral particles

For inducible transgene overexpression the Lenti-X Tet-ON Advanced Inducible Expression System from Clontech was used. A modified version of the pLVX-Tet-On Advanced vector (Clontech,Cat. No. 632162), which carries the EF1α promoter instead of the original CMV promoter as described by Mertens et al., 2013 and designated here as Eto construct was used as tetracycline/doxycycline-dependable regulator vector. For transgene delivery and expression, genes of interest (e.g. GCNF, GCNF mutDBD, GCNF shRNA, GCNF-VP16, NGN2, miR-30-ctr) were cloned into the multiple cloning site of the doxycycline inducible pLVX-Tight response plasmid carrying a puromycine-resistance gene as selectable marker (pLVX-Tight-Puro, Clontech, Cat. No. 632162). The coding DNA sequence for GCNF/NR6A1 was amplified from lt-NES cell cDNA (Koch et al., 2009) and cloned into the pLVX-Tight-Puro backbone. The amplified sequence matched with the GCNF/NR6A1 transcript variant (ENST00000344523). To generate the GCNF mutDBD variant, C60S, C63S, C112S and C115S were introduced via PCR mutagenesis. For cloning the GCNF-VP16, the GCNF cDNA from pLVX-Tight-GCNF-Puro and the VP16 sequence from the mouse GCNF-VP16 construct (kind gift of Austin Cooney (Cooney et al., 1998)) were amplified from the respective constructs. GCNF mutDBD and GCNF-VP16 were assembled into the pLVX-Tight-Puro backbone using NEBuilder^{®} HiFi DNA Assembly Cloning Kit (New England Biolabs). The pLVX-Tight-NGN2-Puro construct was previously generated as described in Ladewig et al., 2012. For cloning the miR-30: shRNA-ctr hybrid cassette, a scrambled non-targeting oligonucleotide sequence was cloned into the linearized pCAG-miR-30 backbone as described by Paddison et al., 2004 and from there transferred to the pLVX-Tight-Puro plasmid. To generate the GCNF shRNA lentiviral construct, oligonucleotide TGCTGTTGACAGTGAGCGACCCATGTTGATTGAAGATGGATAGTGAAGCCACAGATGTATCC ATCTTCAATCAACATGGGCTGCCTACTGCCTCGGA (SEQ ID NO: 24) selected from the shRNA prediction from Fellmann et al., 2013 were cloned into the optimized version of the miR-30 backbone following the authors' instructions and from there transferred to the pLVX-Tight-Puro plasmid.

Lentiviral particles were generated by using 293 FT (ThermoFischer) packaging cells that were equipped with pMD2.G (envelope plasmid, a gift from Didier Trono (Addgene plasmid # 12259; http://n2t.net/addgene: 12259; RRID:Addgene_12259) and psPAX2 (packaging plasmid, gift from Didier Trono (Addgene plasmid # 12260; http://n2t.net/addgene: 12260 ; RRID:Addgene_12260) and the different transgene carrying pLVX-Tight-Puro plasmids following the protocol described in Roese-Koerner et al., 2016. Host cells, here smNPCs and lt-NES cells, were then treated with the lentiviral particles as described in Roese-Koerner et al., 2016. To that end, smNPCs were first transduced with the pLVX-Eto virus, expressing a G418 resistance gene as selectable marker and thereafter propagated in the presence of G418. Subsequently, pLVX-Eto carrying smNPCs were transduced with the different pLVX-Tight-Puro variants and cultivated with puromycin to select for transduced cells. Stably transduced smNPCs were then used for the experiments as described in the following. Transgene expression was induced by addition of 4 µg/ml doxycycline diluted in the respective cell culture medium.

### Immunofluorescence staining

For immunofluorescence experiments, cells were fixed with 4% paraformaldehyde in PBS for 10 minutes at room temperature. Following fixation, cells were permeabilized with 0.1% Triton-X-100 in PBS for 10 min. Afterwards, cells were incubated in blocking solution (5-10% fetal bovine serum (FBS) in 0.1-0.5% Triton/PBS) for up to 2 h at RT. Primary antibodies were applied in blocking solution diluted to indicated concentrations (see below) for either 2 h at RT or for overnight incubation at 4 °C. Cells were washed twice in PBS before secondary antibodies were applied in blocking solution for 2 h at RT. Cell nuclei were counterstained with DAPI (4',6-diamidino-2-phenylindole, 1:10,000 in PBS), and the cells were finally embedded in Mowiol and covered with a glass cover slip. The following antibodies were used: mouse anti-KI67 (Dianova, 1:100), mouse anti-MAP2a+2b (Sigma, 1:500) mouse anti-Nestin (Biolegend, 1:500), mouse anti-PSD95 (Abcam, 1:500), rabbit anti-SOX2 (Cell Signaling, 1:300), guinea pig anti-Synaptophysin (SYSY 1:1000); rabbit anti-ßIII Tubulin (Biozol, 1:500). Alexa-488, Alex-555 conjugated secondary antibodies were obtained from Invitrogen and used at a dilution of 1:1000.

Image analysis and processing: At least three pictures were taken randomly per condition using 10x or 20x microscope and processed using ImageJ. Cell counting was performed using ImageJ, whereby each picture was first scored for DAPI-labeled nuclei and subsequently for the markers of interest. For each condition 5 images were scored. Neurite length of at least 193 cells was determined using the ImageJ plugin NeuronJ (Meijering et al., 2004). For the cell cluster analysis 3 10x pictures were taken randomly per condition using the Zeiss Apotome. ImageJ was used for cell cluster size estimation and counting of nuclei within each cell cluster. Cell clusters were defined as a group of 5 or more cell nuclei in close proximity to each other, meaning in less than one nucleus diameter distance.

### Western Blot

Protein extraction and Western Blots were performed as described in Roese-Koerner et al., 2016. The following antibodies were used: mouse anti-GCNF (Perseus Proteomics 1:1000) and mouse anti- β-actin (Millipore 1:5000). Protein amount was estimated using the Image Lab software from Bio-Rad. The signal for GCNF was normalized to β-actin signal.

### Calcium Imaging

smNPCs carrying GCNF-VP16 or NGN2 overexpression constructs were seeded onto 96 well plates (black µClear from Greiner) coated with PEI/Laminin. The next day, smNPCs were treated with N2B27 medium + 4 µg/ml doxycycline. This treatment was continued for 1 week with medium change every other day before switching to the maturation medium at day 7, which consists of N2B27 + 10 ng/µl BDNF (Cell Guidance System) Calcium imaging was performed 6 weeks after first day of transgene induction by using the Fluo-4 NW Ca²⁺ assay kit from Invitrogen (Ref# F36206) following manufacturer's instructions. Imaging was performed with the InCell 2000 microscope and analyzed with ImageJ.

### MEA

smNPCs carrying GCNF-VP16 or NGN2 overexpression constructs were seeded onto 24 well MEA plates (CytoView MEA 24) coated with 1:30 Geltrex. The next day, smNPCs were treated with N2B27 medium + 4 µg/ml doxycycline. This treatment was continued for 2 weeks with medium change every other day. At day 14 doxycycline treatment was omitted. Once a week the spontaneous electrophysiological activity was monitored by the Maestro Edge system from Axion Biosystems using the following settings: configuration_neural real time_spontaneous_default settings.

### Flow cytometry

To process samples for flow cytometric (FLC) analysis, adherent cells were first harvested by enzymatic digestion using Accutase (StemPro, Gibco). After washing the cells with PBS, they were incubated with LIVE/DEAD^{™} Fixable Near-IR Dead Cell Stain (1:1000) for 30 minutes at RT in the dark. For surface staining with CD49f and CD200 antibodies, cells were then washed again with PBS, pelleted by centrifugation, resuspended in 2% FCS in PBS and incubated with rat anti-CD49f-PE (ThermoFischer 1:100) and mouse anti-CD200-APC (ThermoFischer 1:100) for 30 minutes at RT in the dark.

For EDU incorporation assay, cell cultures were first incubated for 3 hours with 10 µM EDU (5-ethynyl- 2'-deoxyuridine), harvested and then stained with LIVE/DEAD ^{™} Fixable Near-IR Dead Cell Stain solution as described above. Subsequently, cells were washed, pelleted by centrifugation and resuspended 1% BSA, 2% PFA in PBS for fixation. After 5 minutes incubation on ice, cells were washed, pelleted by centrifugation and resuspended in 1% BSA, 0.25% Triton in PBS and incubated for 10 minutes on ice for permeabilization. Next, cells were stained for 30 minutes at room temperature using the Click-iT EdU Flow Cytometry Cell Proliferation Assay (Invitrogen) to label EDU-positive cells. Subsequently, cells were washed and incubated with 10 µg/ml DAPI and 50 µg/ml in PBS at 37°C for DNA staining.

Stained cells were washed, resuspended in appropriate amounts of PBS and filtered through a nylon mesh (40 µm) into flow cytometry tubes. The samples were analyzed using the BD FACSCanto III flow cytometry system or the BD Accuri C6 Plus Flow Cytometer. Instrument settings were calibrated using control samples for the different staining conditions and cells were gated based on their size (forward scatter, FSC) and granularity (side scatter, SSC), for viability (through the LIVE/DEAD staining) and detected through the specific fluorophore signal. FLC data were analyzed using the FlowJo Software.

### Gene expression analysis by qRT-PCR and microarrav analysis

RNA was isolated using PeqGOLD TriFast (Peqlab) following the manufacturer's instructions, treated with DNAse (Invitrogen) and reverse-transcribed to generate cDNA with iScript cDNA Synthesis Kit (Bio-Rad). Quantitative real-time RT-PCR reactions were performed using Taq Polymerase (Invitrogen) and a self-made SYBR-green-based reaction mix on an Eppendorf Mastercycler. Primers used were: 18s RNA (For TTCCTTGGACCGGCGCAAG (SEQ ID NO: 25), Rev GCCGCATCGCCGGTCGG (SEQ ID NO: 26)), GCNF (For GAGGCCGGAATAAGAGCATT (SEQ ID NO: 27), Rev CAGGGGAACTGTGGTCACTATC (SEQ ID NO: 28)) and BCL11a (For GCAACACGCACAGAACACTC (SEQ ID NO: 29), Rev GAGCTTCCATCCGAAAACTG (SEQ ID NO: 30)). Data were normalized to 18S rRNA levels. PCR products were assessed by dissociation curve and gel electrophoresis. Normalized data were analyzed using the DDCt method.

For microarray-based gene expression analysis, RNA samples were sent to ATLAS Biolabs. RNA integrity was examined on an Agilent 2100 BioAnalyzer (Agilent Technologies). Biotinylated ss-cDNA were prepared according to the standard Affymetrix protocol using the GeneChip WT PLUS Reagent Kit User Manual (Affymetrix/Thermo Fisher Scientific). Fragmented and labeled ss-cDNA were hybridized on a GeneChip Clariom S Array (Affymetrix/ThermoFisher Scientific). Raw data were processed using Affymetrix Expression Console Software. All samples passed QC thresholds for hybridization and labeling. Data were further analyzed for differential gene expression using the Transcriptome Analysis Console Software (TAC 4.0, ThermoFisher Scientific). TAC 4.0 was also used for performing a principal component analysis (PCA) calling the R prcomp function. The variance of the probe sets was displayed with a maximal number of data points of 5000. Gene ontology enrichment was performed using the WebGestalt web tool (Liao et al., 2019) with the following settings: Enrichment Category, *geneontology_Biological_Process_noRedundant;* Minimum number of IDs in the category, 5; Maximum number of IDs in the category, *2000;* False Discovery Rate (FDR), *BH;* Significance level; *FDR* < *0.05;* Redundancy reduction of categories, *weighted set cover;* Reference list: Human *Clariom S transcript list (21448 in total).* Biological process gene ontology annotation of individual genes displayed in Table 1 was performed using DAVID Bioinformatics Resource 6.8 (Huang et al., 2009).

### Chromatin Immunoprecipitation

To facilitate ChIP of GCNF-bound chromatin, GCNF was tagged with an AM-tag from Active Motif. The coding DNA sequence for GCNF/NR6A1 and the sequence of the AM-tag provided by Active Motif (see Active Motif, pAM_1C Empty Vector Cat. No, 52023) were amplified and cloned into the pLVX-Tight-Puro backbone. Lentiviral particle production and smNPC transduction were carried out as described before. smNPCs carrying a GCNF overexpression construct with an N-terminal AM-tag from Active Motifs were cultivated for 48 h in presence of 4 µg/ml doxycycline before the cells were detached and fixed in suspension with 1% formaldehyde. The ChIP assay was performed by using the Tag-ChIP-IT kit from Active Motif and all steps were executed according to the protocol manual. Polymerase II antibody (Active Motif, 5 µg), AM antibody (Active Motif, 10 µg) and IgG isotype control (Santa Cruz, 10 µg) were used to pull down protein-bound DNA sequences. For qPCR after the ChIP, PerfeCTa SYBR Green SuperMix and the negative control primer set 1 from Active Motif for amplifying DNA sequences of a gene desert region of chromosome 12 and self-designed primers for the region of the BCL11A promotor containing the binding site of GCNF were used (For GGGAGAGAGAGAGAGAGAGATGA (SEQ ID NO: 29), Rev TGTCTCTGTCCATCCAGACTC (SEQ ID NO: 30)).

### Statistical analysis

Statistical analysis was performed using GraphPad Prism. All data were first tested for their normality by Shapiro-Wilk test. If the data points passed the normality test, unpaired t-test was performed. If the data sets did not pass the normality test, Mann-Whitney test was performed.

### Results

### Example 1:

The inventors have previously discovered that overexpression of GCNF inhibits neuronal differentiation of hPSC-derived NSCs (Stappert, 2014). It is assumed that GCNF acts as a repressor of target gene expression (Cooney et al., 1998). Thus, the inventors wondered whether addition of the transactivator domain of VP16 to GCNF, which is known to activate GCNF target genes (Barreto et al., 2003; Fuhrmann et al., 2001; Hentschke et al., 2006; Rajkovic et al., 2010; e.g. Yan and Jetten, 2000) would enhance neuronal differentiation. To assess the potential of GCNF-VP 16 on neuronal differentiation the inventors chose smNPCs as cell model, which were transduced with a lentiviral-based doxycycline inducible expression system. smNPCs were either transduced with only the Eto regulator (as control) or co-transduced with the Eto regulator and one of the different transgene constructs to modulate GCNF activity, i.e. GCNF, GCNF shRNA and GCNF-VP16, respectively (Figure 2A). As additional controls, constructs coding for miR-30-ctr (ctr) and a GCNF derivate, in which the DBD was mutated (GCNF mutDBD), were used. As a benchmark, smNPCs carrying an ectopic expression construct for NGN2, which is a known pro-neurogenic factor (Cheng et al., 2017; Ho et al., 2016), were generated. The different transgenic smNPC lines were then differentiated to form neurons, treated with doxycycline to induce transgene expression (Figure 2A) and characterized by immunofluorescence and flow cytometry to assess the number of undifferentiated progenitors versus differentiated neuronal cells.

Under default conditions, the majority of cells (71.5 ± 1.9%) still stains positive for the NPC marker SOX2. While GCNF overexpression results in a slightly increase of SOX2-positve cells (77.2 ± 1.5%), the percentage of SOX2 positive cells drops to 27.5 ± 4.1 % and 54.4 ± 3.7% in GCNF-VP16 and NGN2 cultures, respectively (Figure 2B). GCNF-VP16 and NGN2 overexpression significantly reduced the rate of EDU incorporation used here to assess cell proliferation, whereas GCNF overexpression had the opposite effect (Figure 2C). The negative impact of GCNF-VP16 and NGN2 on cell proliferation is also reflected by the lower cell density of the cultures (Figure 2E). Staining for MAP2 as a marker for neuronal cells revealed that GCNF-VP16 and NGN2 cultures from a network of differentiated cells already after 7 days. While the fraction of MAP2 positive cells with respect to the overall reduced cell number seems to be higher in GCNF-VP16 and NGN2 compared to control lines, a quantification of the number of MAP2 positive cells was not possible. Thus, to assess the amount of undifferentiated versus differentiated cells in smNPC cultures, a flow cytometry-based read-out for CD49f and CD200 expression was used as proxy (Turaç et al., 2012). CD49f (α6-Integrin) has been associated with undifferentiated proliferative neural cells, while CD200 (MOX2) is expressed on the surface of differentiated neuronal cells (Turaç et al., 2012). The percentage of cells expressing CD49f at low and CD200 at high levels, which indicates cells that have embarked on neuronal differentiation, was around 45% in Eto and miR-30-ctr cultures (Figure 2D). This was drastically increased to over 80% of CD49f^{low}/CD200^{high} cells in GCNF-VP16 and NGN2 cultures. The percentage of CD49f^{low}/CD200^{high} cells was increased to 66.7 ± 2.1% upon shRNA mediated knockdown of GCNF, while it was reduced to 29.4 ± 2.5% upon GCNF overexpression (Figure 2D). Taken together, these results confirm the negative impact of GCNF on neuronal differentiation, which was previously observed by the inventors, and demonstrate that knockdown of GCNF or expression of GCNF-VP16 promotes neuronal differentiation of smNPCs. The effect of GCNF-VP 16 on neuronal differentiation appears comparable to that of NGN2 induction.

When differentiating, smNPCs typically form cell clusters with densely packed cell bodies and outward pointing neurites. Interestingly, cell cluster formation appeared to be more prominent in GCNF overexpression cultures, whereas it seemed to be strongly impaired in GCNF-VP 16 cultures (Figure 2E and Figure 3A). In a detailed examination, the cluster size and cell density per cluster was determined. For this analysis a "cell cluster" was defined as a group of at least 5 cells in close proximity to each other, i.e. with less than one nucleus diameter distance (Figure 3B). In miR-30-ctr cultures, the average size of clusters was ~6000 µm² and the average cell number per cluster 54. The cell clusters formed in GCNF overexpressing cultures were much larger (average size 12500 µm²) and contained more cells per cluster (average 144) indicating that they were denser packed. GCNF-VP16 induced neurons formed smaller clusters (∼1400 µm²) containing a lower number of cells (average 14). Both cluster size and number of nuclei per cluster were also reduced in NGN2 cultures but not as much as in GCNF-VP 16 cultures (Figure 3C, D, E). These data demonstrate that GCNF and GCNF-VP16 have a distinct but opposite effect on the cell cluster formation of differentiating smNPCs.

The inventors performed microarray-based transcriptome analysis to monitor expression changes related to neuronal differentiation and transgene expression. To that end, samples from smNPCs transduced with Eto, miR-30-ctr, GCNF, GCNF-VP16 and NGN2, respectively were harvested at 2 (ND2), 7 (ND7) and 14 (ND14) days of neuronal differentiation and subjected to microarray gene expression analysis. Differential gene expression analysis across the different time point revealed 2463 differentially expressed genes between ND2 and ND14 control samples (i.e. Eto and miR-30-ctr transduced smNPCs; Figure 4A). Genes associated with Gene ontology terms "DNA conformation change", "DNA replication", and "mitotic cell phase transition" were found to be down-regulated with differentiation, while genes associated with "positive regulation of neurogenesis", "axon development", and "synapse organization" were found to be up-regulated. Differential gene expression analysis across the different transgenic cultures revealed a profound effect of GCNF-VP16 and NGN2 on gene expression at ND7 with 1449 differentially expressed genes for GCNF-VP16 and 1891 differentially expressed genes for NGN2. GCNF overexpression had a modest effect with only 138 of differentially expressed genes (Figure 4A).
Principal component analysis (PCA) indicates that GCNF-VP 16 samples of all time points are distinct from all other samples (Figure 4B). Interestingly, ND2 samples of GCNF-VP16 and NGN2 appear to be more similar to ND7 control samples pointing to an accelerated acquisition of the differentiation-associated gene signature in GCNF-VP16 and NGN2. In contrast, GCNF ND7 and ND14 samples are shifted to the left on PCA1 axis compared to the respective control counterparts suggesting a delayed transcriptome trajectory. This observation of an altered speed in shifting towards a differentiation gene program is also supported by gene ontology enrichment analysis (Figure 4C, D). Genes linked to gene ontology terms "axon development", "synapse organization" were up-regulated in GCNF-VP16, while genes associated with "NPC proliferation" and "mitotic cell phase transition" were down-regulated compared to controls (Figure 4C, see Table 1 for an overview of functionally-relevant up-regulated genes in GCNF-VP16 ND7). Instead, genes linked to gene ontology term "stem cell differentiation" were still expressed at high levels in GCNF ND7 compared to controls (Figure 4D). In line with the findings on an altered cell clustering, genes associated with "cell-cell adhesion" (e.g. genes coding for protocadherins and cadherins, NRNX1, L1CAM), were found to be up-regulated by GCNF-VP16, whereas genes associated with "extracellular structure organization" (e.g. genes coding for collagen chains, integrin subunits, laminin, fibronectin) and "morphogenesis of an epithelium" (e.g. MET, FGF2, EGFR, WNT5A, WNT7A, SHROOM) were down-regulated.

The inventors identified BCL11A as the most up-regulated gene in GCNF-VP16 after 48 hours of transgene induction (Figure 5A). BCL11A is a transcriptional repressor, which is described to regulate neuronal differentiation and morphogenesis, as well as sensory circuit formation in dorsal spinal cord development (John et al., 2012). Radial migration of upper layer cortical neurons in mice was also found to be affected by BCL11A (Wiegreffe et al., 2015). Interestingly, GCNF and BCL11A show an opposite expression profile during smNPC differentiation: GCNF is down-regulated during differentiation, while BCL11A is upregulated (Figure 5B). The impact of GCNF modulation on BCL11A expression was further validated by qRT-PCR of smNPCs carrying different GCNF modulating lentiviral constructs: Overexpression of GCNF inhibits the expression of BCL11A, while knockdown of GCNF via shRNA or expression of GCNF-VP16 increases BCL11A expression (Figure 5C). Bioinformatic analysis by TRANSFAC Match Tool revealed three different binding sites for GCNF within the BCL11A promotor region (Figure 5D). To confirm binding of GCNF to the BCL11A promotor and prove that BCL11A is a direct target gene of GCNF chromatin immunoprecipitation (ChIP) was performed. There are no ChIP-grade GCNF antibodies available. Thus, the inventors designed a lentiviral construct coding for GCNF with a N-terminal AM-tag (GCNF-AM) to enable precipitation of protein-bound DNA fragments using a ChIP-grade AM-directed antibody (Active Motifs). Chromatin bound by GCNF was pulled down with the AM antibody and the enrichment of GCNF-AM-bound chromatin was analyzed by qPCR using a primer for the region of the BCL11A promotor containing putative GCNF binding sites. The region of the BCL11A promotor was significantly enriched in GCNF-AM precipitate and RNA polymerase II-precipitate compared to the input control (Figure 5E, F).

Next, the inventors analyzed if the generated neurons formed by GCNF-VP16 overexpression were mature and functional active. To that end, smNPCs overexpressing either GCNF-VP16 or NGN2 were differentiated for five weeks and analyzed by immunostainings for Synaptophysin and PSD95. Indeed, in both GCNF-VP16 and NGN2 neurons, Synaptophysin- and PSD95-positive structures were noted indicating the presence of pre- and post-synaptic formations (Figure 6A). Calcium-imaging of 6 weeks-differentiated GCNF-VP16 or NGN2 neurons demonstrated that both neuronal cultures respond to membrane depolarization stimuli (Figure 6B). Multielectrode array (MEA) further showed spontaneous activity of GCNF-VP16 or NGN2 neurons (Figure 6C). Taken together these results suggest that GCNF-VP16 neurons achieve functional maturity in a similar time range as NGN2 neurons.

In order to investigate the effect of GCNF-VP 16 at early stage of neuronal differentiation, smNPCs transduced with miR-30-ctr, GCNF-VP16 or NGN2 constructs were differentiated for 2 days in the presence of doxycycline and analyzed by immunostaining and flow cytometry. In control cultures only 9.2 ± 0.9 % of cells stained positive for the neuronal marker MAP2. In cells overexpressing GCNF-VP16 or NGN2 percentage of MAP2-positive cells was increased to 25.3 ± 5.2 % and 13.4 ± 0.3 %, respectively (Figure 7A, B). While the effect on the number of SOX2-positive cells is only subtle (Figure 7C), GCNF-VP16 and NGN2 overexpression reduced the number of KI67-positive and EDU-positive cells pointing to an inhibitory effect on cell proliferation (Figure 7D, E). Taken together, these results confirm that overexpression of GCNF-VP16 induces a shift from proliferation to neuronal differentiation already after 48 hours of transgene expression. Interestingly, the impact of GCNF-VP16 in the settings applied here appears to be stronger than the effect of NGN2 overexpression.

In summary, the data generated by the inventors demonstrate a positive effect of GCNF-VP16 on neuronal differentiation. GCNF-VP16 cultures show an accelerated shift from proliferation to differentiation and are able to mature into functional-active neurons. Furthermore, they form less clusters, and GCNF-VP16 was found to affect the expression of several cell-adhesion genes. GCNF-VP16 also up-regulates genes associated with neuronal differentiation and maturation, including BCL11A, which was identified as a direct target gene of GCNF. Thus, BCL11A is a likely downstream candidate of GCNF/GCNF-VP16 in regulating neuronal differentiation.

### Example 2:

Lt-NES cells derived from the I3 hES cell line were transduced with lentiviral constructs coding for GCNF cDNA, GCNF-VP16 or a shRNA directed against GCNF. Expression of the GCNF-shRNA construct led to a repression of GCNF levels by at least 50%. The cultures were then differentiated for 7 days in the presence of doxycycline to induce transgene expression, fixed and analyzed by immunofluorescence for expression of NESTIN as neural progenitor marker and β-III Tubulin as neuronal marker (Figure 8A). As for the smNPCs (see Example 1), the overall cell density was decreased in GCNF-VP16 expressing cultures, while the percentage of β-III Tubulin+ neurons was increased as compared to the control culture. Likewise, a reduction in overall cell number and an increased neuronal differentiation were observed in GCNF-shRNA expressing cultures. Lt-NES also differentiate as cell clusters. As noted before for smNPCs, GCNF overexpressing lt-NES cells showed a more prominent cluster formation compared to control cultures and also the number of β-III Tubulin positive neurons was decreased. Flow cytometric readout of the expression levels of CD49f and CD200 were used here as proxy to measure the amount of undifferentiated neural progenitors versus differentiated neurons confirmed that GCNF overexpression leads to a reduced neuronal differentiation, while GCNF-VP16 and GCNF shRNA promote neuronal differentiation (Figure 8B). Treatment with Notch inhibitor DAPT as known neurogenic substance (Borghese et al., 2010) was used as a positive control in this readout.

### REFERENCES

Amit M, Itskovitz-Eldor J. Derivation and spontaneous differentiation of human embryonic stem cells. J Anat. 2002nd ed. 2002 Mar;200(Pt 3):225-32.
Barreto, G., Borgmeyer, U., and Dreyer, C. (2003). The germ cell nuclear factor is required for retinoic acid signaling during Xenopus development. Mechanisms of Development 120, 415-428. doi:10.1016/S0925-4773(03)00018-2.
Borghese, L., Dolezalova, D., Opitz, T., Haupt, S., Leinhaas, A., Steinfarz, B., et al. (2010). Inhibition of Notch signaling in human embryonic stem cell-derived neural stem cells delays G1/S phase transition and accelerates neuronal differentiation in vitro and in vivo. STEM CELLS 28, 955-964. doi: 10.1002/stem.408. Borys S. M., Younger S. T. (2020). Identification of functional regulatory elements in the human genome using pooled CRISPR screens. BMC Genomics Jan, 1-15. doi: 10.1186/s12864-020-6497-0.
Britsch S., Bcl11 Transcription Factors Regulate Cortical Development and Function. fnmol-13-00051tex. 2020 Apr 8;:1-10.
Cheng, C., Fass, D. M., Folz-Donahue, K., MacDonald, M. E., and Haggarty, S. J. (2017). Highly Expandable Human iPS Cell-Derived Neural Progenitor Cells (NPC) and Neurons for Central Nervous System Disease Modeling and High-Throughput Screening. Curr Protoc Hum Genet 92, 21.8.1-21.8.21. doi: 10.1002/cphg.33.
Conti, L., and Cattaneo, E. (2010). Neural stem cell systems: physiological players or in vitro entities? Nat Rev Neurosci 11, 176-187. doi: 10.1038/nrn2761.
Cooney, A. J., Hummelke, G. C., Herman, T., Chen, F., and Jackson, K. J. (1998). Germ cell nuclear factor is a response element-specific repressor of transcription. Biochem. Biophys. Res. Commun. 245, 94-100. doi:10.1006/bbrc.1998.8391.
Darbinyan, A., Kaminski, R., White, M. K., Darbinian, N., and Khalili, K. (2013). "Isolation and Propagation of Primary Human and Rodent Embryonic Neural Progenitor Cells and Cortical Neurons," in Transcrip-tional Regulation Methods in Molecular Biology. (Totowa, NJ: Humana Press), 45-54. doi: 10.1007/978-1-
Elkabetz, Y., Panagiotakos, G., Shamy, A1, G., Socci, N. D., Tabar, V., and Studer, L. (2008). Human ES cell-derived neural rosettes reveal a functionally distinct early neural stem cell stage. Genes & Development 22, 152-165. doi: 10.1101/gad.1616208.
Fellmann C, Hoffmann T, Sridhar V, Hopfgartner B, Muhar M, Roth M, et al. An Optimized microRNA Backbone for Effective Single-Copy RNAi. Cell Reports. 2013 Dec;5(6):1704-13.
Fuhrmann, G., Chung, A. C., Jackson, K. J., Hummelke, G., Baniahmad, A., Sutter, J., et al. (2001). Mouse Germline Restriction of Oct4 Expression by Germ Cell Nuclear Factor. Dev. Cell 1, 11-11.
Futaki S., Goto S., Suzuki T., Nakase I., Sugiura Y. (2003). Structural variety of membrane permeable peptides. Curr Protein Pept Sci 4, 87-96. doi: 10.2174/1389203033487261.
Gage, F. H. (2000). Mammalian Neural Stem Cells. Science 287, 1433-1438. doi: 10.1126/science.287.5457.1433.
Gorris, R., Fischer, J., Erwes, K. L., Kesavan, J., Peterson, D. A., Alexander, M., et al. (2015). Pluripotent stem cell-derived radial glia-like cells as stable intermediate for efficient generation of human oligodendrocytes. Glia 63, 2152-2167. doi: 10.1002/glia.22882.
Gu, P., Le Menuet, D., Chung, A. C. K., Mancini, M., Wheeler, D. A., and Cooney, A. J. (2005a). Orphan Nuclear Receptor GCNF Is Required for the Repression of Pluripotency Genes during Retinoic Acid-Induced Embryonic Stem Cell Differentiation. Molecular and Cellular Biology 25, 8507-8519. doi: 10.1128/MCB.25.19.8507-8519.2005.
Gu P., Goodwin B., Chung A.C.K., Xu X., Wheeler D. A, Price R. R., et al. (2005b) Orphan Nuclear Receptor LRH-1 Is Required To Maintain Oct4 Expression at the Epiblast Stage of Embryonic Development. Molecular and Cellular Biology 25, 3492-505. doi: 10.1128/MCB.25.9.3492-3505.2005.
Gu P., Morgan D. H., Sattar M., Xu X., Wagner R., Raviscioni M., et al. (2005c) Evolutionary trace-based peptides identify a novel asymmetric interaction that mediates oligomerization in nuclear receptors. J Biol Chem. American Society for Biochemistry and Molecular Biology 280, 31818-31829. doi:10.1074/jbc.M501924200.
Gu, P., Xu, X., Le Menuet, D., Chung, A. C. K., and Cooney, A. J. (2011). Differential Recruitment of Methyl CpG-Binding Domain Factors and DNA Methyltransferases by the Orphan Receptor Germ Cell Nuclear Factor Initiates the Repression and Silencing of Oct4. STEM CELLS 29, 1041-1051. doi:10.1002/stem.652.
Hentschke, M., Kurth, I., Borgmeyer, U., and Hübner, C. A. (2006). Germ cell nuclear factor is a repressor of CRIPTO-1 and CRIPTO-3. J Biol Chem 281, 33497-33504. doi:10.1074/jbc.M606975200.
Ho, S.-M., Hartley, B. J., TCW, J., Beaumont, M., Stafford, K., Slesinger, P. A., et al. (2016). Rapid Ngn2-induction of excitatory neurons from hiPSC-derived neural progenitor cells. Methods 101, 113-124. doi:10.1016/j.ymeth.2015.11.019.
Huang D. W., Sherman B. T., Lempicki R. A. (2009). Systematic and integrative analysis of large gene lists using DAVID bioinformatics resources. Nat Protoc 4, 44-57. doi:10.1038/nprot.2008.211
Irion, S., Luche, H., Gadue, P., Fehling, H. J., Kennedy, M., and Keller, G. (2007). Identification and targeting of the ROSA26 locus in human embryonic stem cells. Nat Biotechnol 25, 1477-1482. doi:10.1038/nbt1362.
Irion, S., Zabierowski, S. E., and Tomishima, M. J. (2017). Bringing Neural Cell Therapies to the Clinic: Past and Future Strategies. Molecular Therapy: Methods & Clinical Development 4, 72-82. doi:10.1016/j.omtm.2016.11.005.
John A., Brylka H., Wiegreffe C., Simon R., Liu P., Juttner R., et al. (2012). Bel11a is required for neuronal morphogenesis and sensory circuit formation in dorsal spinal cord development. Development 139, 1831-1841.doi:10.1242/dev.072850.
Karus, M., Blaess, S., and Briistle, O. (2014). Self-organisation of neural tissue architectures from pluripotent stem cells. J. Comp. Neurol. doi:10.1002/cne.23608.
Kiani, S., Chavez, A., Tuttle, M., Hall, R.N., Chari, R., Ter-Ovanesyan, D., et al., (2015). Cas9 gRNA engineering for genome editing, activation and repression. Nat Methods 12(11): 1051-1054. doi: 10.10/38/nmeth.3580
Koch, P., Opitz, T., Steinbeck, J. A., Ladewig, J., and Brüstle, O. (2009). A rosette-type, self-renewing human ES cell-derived neural stem cell with potential for in vitro instruction and synaptic integration. Proc Natl Acad Sci U S A 106, 3225-3230. doi:10.1073/pnas.0808387106.
Koziollek-Drechsler, I., SATTLER, U., and Zechel, C. (2005). The expression level of GCNF affects fate choice during neural differentiation of PCC7 cells. Signal Transduction 5, 48-54. doi:10.1002/sita.200400041.
Kumar, A., Declercq, J., Eggermont, K., Agirre, X., Prosper, F., and Verfaillie, C. M. (2012). Zic3 induces conversion of human fibroblasts to stable neural progenitor-like cells. Journal of Molecular Cell Biology 4, 252-255. doi:10.1093/jmcb/mjs015.
Ladewig, J., Mertens, J., Kesavan, J., Doerr, J., Poppe, D., Glaue, F., et al. (2012). Small molecules enable highly efficient neuronal conversion of human fibroblasts. Nat Meth 9, 575-578. doi:10.1038/nmeth.1972.
Lee, J. H., Mitchell, R. R., McNicol, J. D., Shapovalova, Z., Laronde, S., Tanasijevic, B., et al. (2015). Single transcription factor conversion of human blood fate to NPCs with CNS and PNS Developmental Capacity. CellReports 11, 1367-1376. doi:10.1016/j.celrep.2015.04.056.
Li, W., Sun, W., Zhang, Y., Wei, W., Ambasudhan, R., Xia, P., et al. (2011). Rapid induction and long-term self-renewal of primitive neural precursors from human embryonic stem cells by small molecule inhibi-tors. Proc Natl Acad Sci U S A 108, 8299-8304. doi:10.1073/pnas.1014041108.
Liao Y., Wang J., Jaehnig EJ., Shi Z., Zhang B. (2019). WebGestalt 2019: gene set analysis toolkit with revamped UIs and APIs. Nucleic Acids Research. Oxford University Press 47, 199-205. doi: 10.1242/dev.072850.
Martínez-Cerdeño, V., and Noctor, S. C. (2018). Neural Progenitor Cell Terminology. Front. Neuroanat. 12, 664-8. doi: 10.3389/fnana.2018.00104.
McCarty N. S., Graham A. E., Studená L., Ledesma-Amaro R. (2020). Multiplexed CRISPR technologies for gene editing and transcriptional regulation. Nat Comms. Springer US 2020, 1-13.doi: 10.1038/s41467-020-15053-x.
Mitchell, R. R., Szabo, E., Benoit, Y. D., Case, D. T., Mechael, R., Alamilla, J., et al. (2014). Activation of Neural Cell Fate Programs Toward Direct Conversion of Adult Human Fibroblasts into Tri-Potent Neural Progenitors Using OCT-4. Stem Cells Dev 23, 1937-1946. doi:10.1089/scd.2014.0023.
Okumura, L. M., Lesch, B. J., and Page, D. C. (2013). The Ligand Binding Domain of GCNF Is Not Required for Repression of Pluripotency Genes in Mouse Fetal Ovarian Germ Cells. PLoS ONE 8, e66062. doi:10.1371journal.pone.0066062.g007.
Paddison, P. J., Cleary, M., Silva, J. M., Chang, K., Sheth, N., Sachidanandam, R., et al. (2004). Cloning of short hairpin RNAs for gene knockdown in mammalian cells. Nat Meth 1, 163-167. doi: 10.1038/nmeth1104-163.
Papapetrou, E. P., and Schambach, A. (2016). Gene Insertion Into Genomic Safe Harbors for Human Gene Therapy. Molecular Therapy 24, 678-684. doi:10.1038/mt.2016.38.
Pandelakis, M., Delgado, E., Ebrahimkhani, M. R. (2020). CRISPR-Based Synthetic Transcription Factors In Vivo: The Future of Therapeutic Cellular Programming. Cell Systems 10, doi.org/10.1016/j.cels.2019.10.003.
Park S-W., Do H-J., Choi W., Kim J-H., Song H., Seo H. G., et al. (2017). GCNF regulates OCT4 expression through its interactions with nuclear receptor binding elements in NCCIT cells. J Cell Biochem 33, 1-12. doi:0.1002/jcb.26438.
Pawlowski, M., Ortmann, D., Bertero, A., Tavares, J. M., Pedersen, R. A., Vallier, L., et al. (2017). Inducible and Deterministic Forward Programming of Human Pluripotent Stem Cells into Neurons, Skeletal Myocytes, and Oligodendrocytes. Stem Cell Reports 8, 803-812. doi:10.1016/j.stemcr.2017.02.016.
Pollard, S. M., Conti, L., Sun, Y., Goffredo, D., and Smith, A. (2006). Adherent Neural Stem (NS) Cells from Fetal and Adult Forebrain. Cereb. Cortex 16, i112-i120. doi:10.1093/cercor/bhj167.
Qi, Y., Zhang, X.-J., Renier, N., Wu, Z., Atkin, T., Sun, Z., et al. (2017). Combined small-molecule inhibition accelerates the derivation of functional cortical neurons from human pluripotent stem cells. Nature Publishing Group, 1-13. doi:10.1038/nbt.3777.
Qian, K., Huang, C.-L., Chen, H., Blackbourn, L. W., IV, Chen, Y., Cao, J., et al. (2014). A Simple and Efficient System for Regulating Gene Expression in Human Pluripotent Stem Cells and Derivatives. STEM CELLS 32, 1230-1238. doi:10.1002/stem.1653.
Rajkovic, M., Iwen, K. A. H., Hofmann, P. J., Harneit, A., and Weitzel, J. M. (2010). Functional cooperation between CREM and GCNF directs gene expression in haploid male germ cells. Nucleic Acids Research 38, 2268-2278. doi: 10.1093/nar/gkp1220.
Rajkovic, M., Middendorff, R., Wetzel, M. G., Frkovic, D., Damerow, S., Seitz, H. J., et al. (2004). Germ cell nuclear factor relieves cAMP-response element modulator tau-mediated activation of the testis-specific promoter of human mitochondrial glycerol-3-phosphate dehydrogenase. Journal of Biological Chemistry 279, 52493-52499. doi:10.1074/jbc.M404467200.
Reinhardt, P., Glatza, M., Hemmer, K., Tsytsyura, Y., Thiel, C. S., Höing, S., et al. (2013). Derivation and expansion using only small molecules of human neural progenitors for neurodegenerative disease modeling. PLoS ONE 8, e59252. doi:10.1371/journal.pone.0059252.s013.
Roese-Koerner, B., Stappert, L., Berger, T., Braun, N. C., Veltel, M., Jungverdorben, J., et al. (2016). Reciprocal Regulation between Bifunctional miR-9/9(*) and its Transcriptional Modulator Notch in Human Neural Stem Cell Self-Renewal and Differentiation. Stem Cell Reports 7, 207-219. doi:10.1016/j.stemer.2016.06.008.
Sattler, U., Samochocki, M., Maelicke, A., and Zechel, C. (2004). The expression level of the orphan nuclear receptor GCNF (germ cell nuclear factor) is critical for neuronal differentiation. Molecular Endocrinology 18, 2714-2726. doi:10.1210/me.2004-0251.
Sheng, C., Jungverdorben, J., Wiethoff, H., Lin, Q., Flitsch, L. J., Eckert, D., et al. (2018). A stably self-renewing adult blood-derived induced neural stem cell exhibiting patternability and epigenetic rejuvenation. Nat Comms, 1-15. doi:10.1038/s41467-018-06398-5.
Shi, Y., Kirwan, P., Smith, J., Robinson, H. P. C., and Livesey, F. J. (2012). Human cerebral cortex development from pluripotent stem cells to functional excitatory synapses. Nat Neurosci 15, 477-86- S1. doi:10.1038/nn.3041.
Simon R., Wiegreffe C. and Britsch S. (2020). Bcl11 Transcription Factors Regulate Cortical Development and Function. Front. Mol. Neurosci. 13:51. doi: 10.3389/fnmol.2020.00051
Stappert, L (2014). MicroRNAs and orphan nuclear receptor GCNF as novel regulators of human neural stem cell differentiation and neuronal subtype specification. (Doctoral dissertation, Rheinische Friedrich-Wilhelms-Universität Bonn).
Su, X., Kameoka, S., Lentz, S., and Majumder, S. (2004). Activation of REST/NRSF target genes in neural stem cells is sufficient to cause neuronal differentiation. Molecular and Cellular Biology 24, 8018-8025. doi:10.1128/MCB.24.18.8018-8025.2004.
Sung B., Do H-J., Park S-W., Huh S-H., Oh J-H., Chung H-J., et al. (2012). Regulation of OCT4 gene expression by liver receptor homolog-1 in human embryonic carcinoma cells. Biochemical and Biophysical Research Communications 427, 315-220. doi:10.1016/j.bbrc.2012.09.049.
Thier, M.-C., Hommerding, O., Panten, J., Pinna, R., García-González, D., Berger, T., et al. (2019). Identification of Embryonic Neural Plate Border Stem Cells and Their Generation by Direct Reprogramming from Adult Human Blood Cells. Stem Cell 24, 166-182.e13. doi:10.1016/j.stem.2018.11.015.
Turaç, G., Hindley, C. J., Thomas, R., Davis, J. A., Deleidi, M., Gasser, T., et al. (2012). Combined flow cytometric analysis of surface and intracellular antigens reveals surface molecule markers of human neuropoiesis. PLoS ONE 8, e68519-e68519. doi: 10.1371/journal.pone.0068519.
Uemura S., Rothbard J. B., Matsushita H, Tsao P .S,. Fathman C. G., Cooke J. P. (2002). Short polymers of arginine rapidly translocate into vascular cells: effects on nitric oxide synthesis. Circ J. 12, 1155-1160. doi:10.1253/circj.66.1155.
Wagner, R. T., and Cooney, A. J. (2013). Minireview: The Diverse Roles of Nuclear Receptors in the Regulation of Embryonic Stem Cell Pluripotency. Molecular Endocrinology 27, 864-878. doi: 10.1210/me.2012-1383.
Wang, C., Ward, M. E., Chen, R., Liu, K., Tracy, T. E., Chen, X., et al. (2017a). Scalable Production of iPSC-Derived Human Neurons to Identify Tau-Lowering Compounds by High-Content Screening. Stem Cell Reports 9, 1221-1233. doi:10.1016/j.stemcr.2017.08.019.
Wang, C., Ward, M. E., Chen, R., Liu, K., Tracy, T. E., Chen, X., et al. (2017b). Scalable Production of iPSC-Derived Human Neurons to Identify Tau-Lowering Compounds by High-Content Screening. Stem Cell Reports 9, 1221-1233. doi:10.1016/j.stemcr.2017.08.019.
Wang, Q., and Cooney, A. J. (2013). Revisiting the role of GCNF in embryonic development. Seminars in Cell & Developmental Biology. doi:10.1016/j.semcdb.2013.08.003.
Weikum, E. R., Tuntland, M. L., Murphy, M. N., and Ortlund, E. A. (2016). A Structural Investigation into Oct4 Regulation by Orphan Nuclear Receptors, Germ Cell Nuclear Factor (GCNF), and Liver Receptor Homolog-1 (LRH-1). Journal of Molecular Biology 428, 4981-4992. doi:10.1016/j.jmb.2016.10.025.
Wender P. A., Mitchell D. J., Pattabiraman K., Pelkey ET., Steinman L., Rothbard J. B. (2000). The design, synthesis, and evaluation of molecules that enable or enhance cellular uptake: Peptoid molecular transporters. Proc Natl Acad Sci USA. 97, 13003-13008. doi: 10.1073/pnas.97.24.13003.
Wiegreffe C., Simon R., Peschkes K., Kling C., Strehle M., Cheng J., et al. (2015). Bcl11a (Ctip1) Controls Migration of Cortical Projection Neurons through Regulation of Sema3c. Neuron. Elsevier 87, 311-325. doi:10.1016/j.neuron.2015.06.023.
Yan, Z. H., Medvedev, A., Hirose, T., Gotoh, H., and Jetten, A. M. (1997). Characterization of the response element and DNA binding properties of the nuclear orphan receptor germ cell nuclear factor/retinoid receptor-related testis-associated receptor. J Biol Chem 272, 10565-10572. Available at: http://www.ncbi.nlm.nih.gov/entrez/query.fcgi?cmd=Retrieve&db= PubMed&dopt =Citation&list_uids=9099702.
Yan, Z., and Jetten, A. M. (2000). Characterization of the repressor function of the nuclear orphan receptor retinoid receptor-related testis-associated receptor/germ cell nuclear factor. J Biol Chem 275, 35077-35085. doi:10.1074/jbc.M005566200.
Yang H-M., Do H-J., Kim D-K., Park J-K., Chang W-K., Chung H. M., et al. (2007). Transcriptional regulation of human Oct4 by steroidogenic factor-1. J Cell Biochem 101, 1198-209. doi:10.1002/jcb.21244.
Yu, H.-B., Johnson, R., Kunarso, G., and Stanton, L. W. (2011). Coassembly of REST and its cofactors at sites of gene repression in embryonic stem cells. Genome Research 21, 1284-1293. doi:10.1101/gr.114488.110.
Zechel, C. (2005). The germ cell nuclear factor (GCNF). Mol. Reprod. Dev. 72, 550-556. doi:10.1002/mrd.20377.
Zhang, J., and Jiao, J. (2015). Molecular Biomarkers for Embryonic and Adult Neural Stem Cell and Neurogenesis. BioMed Research International 2015, 1-14. doi:10.1155/2015/727542.
Zhang, Y., Pak, C., Han, Y., Ahlenius, H., Zhang, Z., Chanda, S., et al. (2013). Rapid Single-Step Induction of Functional Neurons from Human Pluripotent Stem Cells. Neuron 78, 785-798. doi:10.1016/j.neuron.2013.05.029.

## Claims

1. A method of converting one or more neural cells (NCs) into one or more induced neuronal cells, comprising
a. providing NCs; and
b. contacting the NCs with a transcription factor (TF) or expressing the TF within the NCs, wherein said TF comprises a DNA binding moiety and a transactivation domain (TAD), preferably a heterologous TAD, wherein the DNA binding moiety specifically binds to a direct repeat with zero spacing (DRO) response element;
thereby producing induced neuronal cells.

2. The method of claim 1, wherein the DR0 comprises or consists of the following nucleotide sequence:
N₁N₂N₃N₄N₅AAGN₆N₇N₈N₉ (SEQ ID NO: 21),
wherein N₁ is selected from A, C and T, N₂ is selected from G, T, and A, N₃ is selected from G and T, N₄ is selected from T and C, N₅ is selected from C and T, N₆ is selected from G and T, N₇ is selected from T, C and A, N₈ is selected from C, A, G and T, and N₉ is selected from A, T, and C; in particular N₁ is A, and/or N₂ is G, and/or N₃ is selected from G and T, and/or N₄ is T, and/or N₅ is C, and/or N₆ is selected from G and T, and/or N₇ is selected from T and C, and/or N₈ is selected from C and A, and N₉ is A; preferably wherein the DR0 comprises or consists of the following nucleotide sequence:
AGkTCAAGkTCA (SEQ ID NO: 1),
and its complement, wherein k is in each case independently selected from G or T.

3. The method of any of claims 1 or 2, wherein the DNA binding moiety is a DNA binding domain (DBD) comprising or consisting of a DBD of a TF selected from SF1/NR5A1, LRH-1/NR5A2 and Germ Cell Nuclear Factor (GCNF/NR6A1), optionally comprising amino acid modifications that do not abolish binding to DR0 response elements or enhance binding to DR0 response elements.

4. The method of claim 3, wherein the DBD comprises or consists of a DBD of GCNF, preferably human GCNF, more preferably the DBD comprises or consists of SEQ ID NO: 2.

5. The method of any of claims 1 to 4, wherein the TAD comprises or consists of a TAD selected from the group consisting of the activation domain of a nuclear receptor, in particular of a steroid, thyroid or retinoid acid receptor; a synthetic or chimeric activation domain; a basic or acidic amino acid activation domain; a viral activation domain; an activation domain of the nine-amino-acid TAD (9aaTAD) family, in particular a 9aaTAD as comprised in Gal4, Oaf1, Pip2, Pdr1, Pdr3, Leu3, Teal, Pho4, Gln3, Gcn4, Msn2, Msn4, Rtg3, CREB, CREBaB6, E2A, MLL, p53-TADI, p53-TADII, FOXO3, NF-kB, NFAT, CEBPA/E, ESX, ELF3, ETV1, KLF2/4, EBNA2, VP16, HSF1, HSF2, HsfA, Gli3, Sox18, PIF, Dreb2a, MTF1, OREB1, WRKY45, NS1, MKL1, VP16, EBNA2, KBP220, ECapLL, P201, AH, or B42; a glutamine-rich activation domain, in particular as present in POU2F1 (Oct1), POU2F2 (Oct2) and Sp1); a proline-rich activation domain, in particular as present in c-jun, AP2 or Oct2; an isoleucine-rich activation domain, in particular as present in NTF-1; a Tat activation domain; a BP64 activation domain; a TAF-1 activation domain; a TAF-2 activation domain; a TAU-1 activation domain; a TAU-2 activation domain; a YAP activation domain, a bHLH protein activation domain, in particular as present in MYOD, ASCL1 or NGN2, or a fragment of said activation domains or a modified version thereof, preferably the TAD comprises or consists of the TAD of VP16, VP64, YAP or MYOD, more preferably the TAD comprises or consists of the TAD of VP16, even more preferably the TAD comprises or consists of SEQ ID NO: 5.

6. The method of any of claims 1 to 5, wherein the NCs are neural stem cells (NSCs) or neural progenitor cells (NPCs), in particular human embryonic stem (hES) cell-derived NPCs/NSCs, human induced pluripotent cell (hiPSC)-derived NPCs/NSCs, transient NPCs generated by neural induction of hPSCs, induced neural stem cells (iNSCs) generated from fibroblasts or blood cells, primary human neural progenitor cells; or a cell line with neurogenic differentiation capacity, in particular a cell line selected from L-Myc, ReNcell CX, LUHMES, N2TD2 and SH-SY5Y, preferably the NCs are human NSCs generated from a human induced pluripotent stem cell (hiPSC-NSCs).

7. The method of any of claims 1 to 6, wherein the transcription factor comprises or consists of a polypeptide having the amino acid sequence according to SEQ ID NO: 8 or exhibiting at least 85%, at least 90%, at least 95%, at least 97% or at least 99% sequence identity to SEQ ID NO: 8 and having a transactivation activity of at least 2-fold, particularly at least 5-fold, more particularly at least 10-fold induction of the expression of a gene selected from the group consisting of ZIC3, NFIB, BCL11A, NEUROD1, and NEUROG2.

8. A method of converting one or more NCs into one or more induced neuronal cells, comprising
a. providing NCs; and
b. inhibiting the expression of GCNF in the NCs;
thereby producing induced neuronal cells.

9. A method of converting one or more NCs into one or more induced neuronal cells, comprising
a. providing NCs; and
b. expressing a GCNF target gene in the NCs or contacting the NCs with the gene product of a GCNF target gene;
thereby producing induced neuronal cells,
wherein preferably the GCNF target gene is selected from the group consisting of BCL11A, NEUROD1, and NEUROG2.

10. The method according to claim 9, wherein the GCNF target gene is BCLA11.

11. The method of any of claims 1 to 10, wherein
i) the induced neuronal cell in comparison to the NC is **characterized by**:
a. increased expression of a neuronal marker selected from the group consisting of DCX, βIII-Tubulin (TUBB3), MAP2, Synaptophysin (SYP), PSD95 (DLG4) and CD200;
b. decreased expression of a undifferentiated NC marker selected from the group consisting of Nestin (NES), SOX1, SOX2, PAX2, PAX6, DACH1, PLZF, HES1, GPM6A, RFX4, POU3F3 and CD49f (ITGA6);
c. decreased expression of proliferation markers selected from the group consisting of MKI67, PCNA and CylinD1 (CCND1), CyclinE1 (CCNE1);
d. increased expression of genes of gene ontology classification GO: 0010975~regulation of neuron projection development, GO:0061564~axon development, GO:0050808~synapse organization, GO:0099177~trans-synaptic signaling, GO: 00423 91-regulation of membrane potential, GO:0030900~forebrain development, GO:0098742∼cell-cell adhesion via plasma-membrane adhesion molecules;
e. decreased expression of genes of gene ontology classification GO:006131∼neural precursor cell proliferation, GO:0044772∼mitotic cell cycle phase transition, GO:0043062∼extracellular structure organization, GO:0002009~morphogenesis of an epithelium;
f. increased formation of neurite processes;
g. increased neurite length; and/or
h. increased calcium influx in response to membrane depolarization; and/or
ii) a cell culture comprising or consisting of a plurality of said induced neuronal cells is **characterized by** reduced cell cluster formation, in particular wherein
a. the size of clusters is reduced and/or
b. the number of clusters with a size of above 4000 µm² is reduced by at least 50%, at least 60%, at least 70%, at least 75%, or at least 80%.

12. A TF comprising a DNA binding moiety and a heterologous TAD, wherein the DNA binding moiety specifically binds to a direct repeat with zero spacing (DRO) response element.

13. A nucleic acid encoding the TF of claim 12 or a vector comprising said nucleic acid.

14. A cell comprising the TF of claim 12, the nucleic acid of claim 13 and/or the vector of claim 13, or a cell produced by the method of any of claims 1-11.

15. Use of a cell according to claim 14 in a method of drug screening or as a disease model.

16. A cell according to claim 14 for use in a method of treatment or prevention of a neurological condition, in particular neuronal damage, neuronal loss or a neurodegenerative disease.


SEQUENCE LISTING

<110>  Life & Brain GmbH

       Rheinische Friedrich-Wilhelms-Universität Bonn

<120>  RAPID PRODUCTION OF HUMAN NEURONAL CULTURES WITH SINGLE CELL
       RESOLUTION

<130>  456-27 EP

<160>  34

<170>  PatentIn version 3.5

<210>  1
<211>  12
<212>  DNA
<213>  Artificial Sequence

<220>
<223>  consensus sequence


<220>
<221>  k
<222>  (3)..(3)
<223>  k is independently selected from G or T

<220>
<221>  k
<222>  (8)..(8)
<223>  k is independently selected from G or T

<400>  1
agktcaagkt ca                                                          12


<210>  2
<211>  66
<212>  PRT
<213>  Homo sapiens

<400>  2

Cys Leu Ile Cys Gly Asp Arg Ala Thr Gly Leu His Tyr Gly Ile Ile
1               5                   10                  15


Ser Cys Glu Gly Cys Lys Gly Phe Phe Lys Arg Ser Ile Cys Asn Lys
            20                  25                  30


Arg Val Tyr Arg Cys Ser Arg Asp Lys Asn Cys Val Met Ser Arg Lys
        35                  40                  45


Gln Arg Asn Arg Cys Gln Tyr Cys Arg Leu Leu Lys Cys Leu Gln Met
    50                  55                  60


Gly Met
65

<210>  3
<211>  86
<212>  PRT
<213>  Homo sapiens

<400>  3

Cys Leu Ile Cys Gly Asp Arg Ala Thr Gly Leu His Tyr Gly Ile Ile
1               5                   10                  15

Ser Cys Glu Gly Cys Lys Gly Phe Phe Lys Arg Ser Ile Cys Asn Lys
            20                  25                  30

Arg Val Tyr Arg Cys Ser Arg Asp Lys Asn Cys Val Met Ser Arg Lys
        35                  40                  45

Gln Arg Asn Arg Cys Gln Tyr Cys Arg Leu Leu Lys Cys Leu Gln Met
    50                  55                  60

Gly Met Asn Arg Lys Ala Ile Arg Glu Asp Gly Met Pro Gly Gly Arg
65                  70                  75                  80

Asn Lys Ser Ile Gly Pro
                85


<210>  4
<211>  89
<212>  PRT
<213>  Homo sapiens

<400>  4

Gln Arg Thr Cys Leu Ile Cys Gly Asp Arg Ala Thr Gly Leu His Tyr
1               5                   10                  15

Gly Ile Ile Ser Cys Glu Gly Cys Lys Gly Phe Phe Lys Arg Ser Ile
            20                  25                  30

Cys Asn Lys Arg Val Tyr Arg Cys Ser Arg Asp Lys Asn Cys Val Met
        35                  40                  45

Ser Arg Lys Gln Arg Asn Arg Cys Gln Tyr Cys Arg Leu Leu Lys Cys
    50                  55                  60

Leu Gln Met Gly Met Asn Arg Lys Ala Ile Arg Glu Asp Gly Met Pro
65                  70                  75                  80

Gly Gly Arg Asn Lys Ser Ile Gly Pro
                85

<210>  5
<211>  23
<212>  PRT
<213>  Artificial Sequence

<220>
<223>  of viral origin

<400>  5

Leu Asp Met Ala Asp Phe Glu Phe Glu Gln Met Phe Thr Asp Ala Leu
1               5                   10                  15

Gly Ile Asp Glu Tyr Gly Gly
            20

<210>  6
<211>  35
<212>  PRT
<213>  Artificial Sequence

<220>
<223>  of viral origin

<400>  6

Gly Phe Thr Pro His Asp Ser Ala Pro Tyr Gly Ala Leu Asp Met Ala
1               5                   10                  15

Asp Phe Glu Phe Glu Gln Met Phe Thr Asp Ala Leu Gly Ile Asp Glu
            20                  25                  30

Tyr Gly Gly
        35

<210>  7
<211>  80
<212>  PRT
<213>  Artificial Sequence

<220>
<223>  of viral origin

<400>  7

Ser Thr Ala Pro Pro Thr Asp Val Ser Leu Gly Asp Glu Leu His Leu
1               5                   10                  15

Asp Gly Glu Asp Val Ala Met Ala His Ala Asp Ala Leu Asp Asp Phe
            20                  25                  30

Asp Leu Asp Met Leu Gly Asp Gly Asp Ser Pro Gly Pro Gly Phe Thr
        35                  40                  45

Pro His Asp Ser Ala Pro Tyr Gly Ala Leu Asp Met Ala Asp Phe Glu
    50                  55                  60

Phe Glu Gln Met Phe Thr Asp Ala Leu Gly Ile Asp Glu Tyr Gly Gly
65                  70                  75                  80

<210>  8
<211>  557
<212>  PRT
<213>  Artificial Sequence

<220>
<223>  fusion construct

<400>  8

Met Glu Arg Asp Glu Pro Pro Pro Ser Gly Gly Gly Gly Gly Gly Gly
1               5                   10                  15

Ser Ala Gly Phe Leu Glu Pro Pro Ala Ala Leu Pro Pro Pro Pro Arg
            20                  25                  30

Asn Gly Phe Cys Gln Asp Glu Leu Ala Glu Leu Asp Pro Gly Thr Ile
        35                  40                  45

Ser Val Ser Asp Asp Arg Ala Glu Gln Arg Thr Cys Leu Ile Cys Gly
    50                  55                  60

Asp Arg Ala Thr Gly Leu His Tyr Gly Ile Ile Ser Cys Glu Gly Cys
65                  70                  75                  80

Lys Gly Phe Phe Lys Arg Ser Ile Cys Asn Lys Arg Val Tyr Arg Cys
                85                  90                  95

Ser Arg Asp Lys Asn Cys Val Met Ser Arg Lys Gln Arg Asn Arg Cys
            100                 105                 110

Gln Tyr Cys Arg Leu Leu Lys Cys Leu Gln Met Gly Met Asn Arg Lys
        115                 120                 125

Ala Ile Arg Glu Asp Gly Met Pro Gly Gly Arg Asn Lys Ser Ile Gly
    130                 135                 140

Pro Val Gln Ile Ser Glu Glu Glu Ile Glu Arg Ile Met Ser Gly Gln
145                 150                 155                 160

Glu Phe Glu Glu Glu Ala Asn His Trp Ser Asn His Gly Asp Ser Asp
                165                 170                 175

His Ser Ser Pro Gly Asn Arg Ala Ser Glu Ser Asn Gln Pro Ser Pro

            180                 185                 190

Gly Ser Thr Leu Ser Ser Ser Arg Ser Val Glu Leu Asn Gly Phe Met
        195                 200                 205

Ala Phe Arg Glu Gln Tyr Met Gly Met Ser Val Pro Pro His Tyr Gln
    210                 215                 220

Tyr Ile Pro His Leu Phe Ser Tyr Ser Gly His Ser Pro Leu Leu Pro
225                 230                 235                 240

Gln Gln Ala Arg Ser Leu Asp Pro Gln Ser Tyr Ser Leu Ile His Gln
                245                 250                 255

Leu Leu Ser Ala Glu Asp Leu Glu Pro Leu Gly Thr Pro Met Leu Ile
            260                 265                 270

Glu Asp Gly Tyr Ala Val Thr Gln Ala Glu Leu Phe Ala Leu Leu Cys
        275                 280                 285

Arg Leu Ala Asp Glu Leu Leu Phe Arg Gln Ile Ala Trp Ile Lys Lys
    290                 295                 300

Leu Pro Phe Phe Cys Glu Leu Ser Ile Lys Asp Tyr Thr Cys Leu Leu
305                 310                 315                 320

Ser Ser Thr Trp Gln Glu Leu Ile Leu Leu Ser Ser Leu Thr Val Tyr
                325                 330                 335

Ser Lys Gln Ile Phe Gly Glu Leu Ala Asp Val Thr Ala Lys Tyr Ser
            340                 345                 350

Pro Ser Asp Glu Glu Leu His Arg Phe Ser Asp Glu Gly Met Glu Val
        355                 360                 365

Ile Glu Arg Leu Ile Tyr Leu Tyr His Lys Phe His Gln Leu Lys Val
    370                 375                 380

Ser Asn Glu Glu Tyr Ala Cys Met Lys Ala Ile Asn Phe Leu Asn Gln
385                 390                 395                 400

Asp Ile Arg Gly Leu Thr Ser Ala Ser Gln Leu Glu Gln Leu Asn Lys
                405                 410                 415

Arg Tyr Trp Tyr Ile Cys Gln Asp Phe Thr Glu Tyr Lys Tyr Thr His
            420                 425                 430

Gln Pro Asn Arg Phe Pro Asp Leu Met Met Cys Leu Pro Glu Ile Arg
        435                 440                 445

Tyr Ile Ala Gly Lys Met Val Asn Val Pro Leu Glu Gln Leu Pro Leu
    450                 455                 460

Leu Phe Lys Val Val Leu His Ser Cys Lys Thr Ser Val Gly Lys Glu
465                 470                 475                 480

Ser Thr Ala Pro Pro Thr Asp Val Ser Leu Gly Asp Glu Leu His Leu
                485                 490                 495

Asp Gly Glu Asp Val Ala Met Ala His Ala Asp Ala Leu Asp Gly Phe
            500                 505                 510

Asp Leu Asp Met Leu Gly Asp Gly Asp Ser Pro Gly Pro Gly Phe Thr
        515                 520                 525

Pro His Asp Ser Ala Pro Tyr Gly Ala Leu Asp Met Ala Asp Phe Glu
    530                 535                 540

Phe Glu Gln Met Phe Thr Asp Ala Leu Gly Ile Asp Glu
545                 550                 555

<210>  9
<211>  12
<212>  DNA
<213>  Homo sapiens

<400>  9
aggccaagtt ca                                                          12

<210>  10
<211>  12
<212>  DNA
<213>  Homo sapiens

<400>  10
agttcaaggt aa                                                          12

<210>  11
<211>  12
<212>  DNA
<213>  Homo sapiens

<400>  11
tggtcaagta ct                                                          12

<210>  12
<211>  12
<212>  DNA
<213>  Homo sapiens

<400>  12
cagccaaggt ca                                                          12


<210>  13
<211>  12
<212>  DNA
<213>  Homo sapiens

<400>  13
agtttaaggc ca                                                          12


<210>  14
<211>  12
<212>  DNA
<213>  Homo sapiens

<400>  14
atgtcaaggt ca                                                          12


<210>  15
<211>  12
<212>  DNA
<213>  Homo sapiens

<400>  15
aggtcaaggc ta                                                          12


<210>  16
<211>  12
<212>  DNA
<213>  Homo sapiens

<400>  16
agttcaaggt ca                                                          12


<210>  17
<211>  12
<212>  DNA
<213>  Homo sapiens

<400>  17
aggtcaagtt cc                                                          12


<210>  18
<211>  12
<212>  DNA
<213>  Homo sapiens

<400>  18
aggtcaaggc ga                                                          12


<210>  19
<211>  12
<212>  DNA
<213>  Homo sapiens

<400>  19
agttcaaggc ca                                                          12

<210>  20
<211>  12
<212>  DNA
<213>  Homo sapiens

<400>  20
agttcaagtg cg                                                          12

<210>  21
<211>  12
<212>  DNA
<213>  Artificial Sequence

<220>
<223>  consensus sequence

<220>
<221>  N
<222>  (1)..(1)
<223>  N1 is selected from A, C and T

<220>
<221>  misc_feature
<222>  (1)..(5)
<223>  n is a, c, g, or t

<220>
<221>  N
<222>  (2)..(2)
<223>  N2 is selected from G, T, and A

<220>
<221>  N
<222>  (3)..(3)
<223>  N3 is selected from G and T

<220>
<221>  N
<222>  (4)..(4)
<223>  N4 is selected from T and C

<220>
<221>  N
<222>  (5)..(5)
<223>  N5 is selected from C and T

<220>
<221>  N
<222>  (9)..(9)
<223>  N6 is selected from G and T

<220>
<221>  misc_feature
<222>  (9)..(12)
<223>  n is a, c, g, or t

<220>
<221>  N
<222>  (10)..(10)
<223>  N7 is selected from T, C, G and A

<220>
<221>  N
<222>  (11)..(11)
<223>  N8 is selected from C, A, G and T

<220>
<221>  N
<222>  (12)..(12)
<223>  N9 is selected from A, T, G and C

<400>  21
nnnnnaagnn nn                                                          12


<210>  22
<211>  8
<212>  DNA
<213>  Artificial Sequence

<220>
<223>  composite site

<400>  22
tgacgtca                                                                8


<210>  23
<211>  12
<212>  RNA
<213>  Artificial Sequence

<220>
<223>  complementary sequence

<400>  23
uagccuugac cu                                                          12


<210>  24
<211>  97
<212>  DNA
<213>  Artificial Sequence

<220>
<223>  oligonucleotide for cloning

<400>  24
tgctgttgac agtgagcgac ccatgttgat tgaagatgga tagtgaagcc acagatgtat      60

ccatcttcaa tcaacatggg ctgcctactg cctcgga                               97


<210>  25
<211>  19
<212>  DNA
<213>  Artificial Sequence

<220>

<223>  primer

<400>  25
ttccttggac cggcgcaag                                                    19


<210>  26
<211>  17
<212>  DNA
<213>  Artificial Sequence

<220>
<223>  primer

<400>  26
gccgcatcgc cggtcgg                                                      17


<210>  27
<211>  20
<212>  DNA
<213>  Artificial Sequence

<220>
<223>  primer

<400>  27
gaggccggaa taagagcatt                                                   20


<210>  28
<211>  22
<212>  DNA
<213>  Artificial Sequence

<220>
<223>  primer

<400>  28
caggggaact gtggtcacta tc                                                22


<210>  29
<211>  20
<212>  DNA
<213>  Artificial Sequence

<220>
<223>  primer

<400>  29
gcaacacgca cagaacactc                                                   20


<210>  30
<211>  20
<212>  DNA
<213>  Artificial Sequence

<220>
<223>  primer

<400>  30

gagcttccat ccgaaaactg                                                    20

<210>  31
<211>  567
<212>  PRT
<213>  Artificial Sequence

<220>
<223>  fusion construct

<400>  31

Met Glu Arg Asp Glu Pro Pro Pro Ser Gly Gly Gly Gly Gly Gly Gly
1               5                   10                  15

Ser Ala Gly Phe Leu Glu Pro Pro Ala Ala Leu Pro Pro Pro Pro Arg
            20                  25                  30

Asn Gly Phe Cys Gln Asp Glu Leu Ala Glu Leu Asp Pro Gly Thr Ile
        35                  40                  45

Ser Val Ser Asp Asp Arg Ala Glu Gln Arg Thr Cys Leu Ile Cys Gly
    50                  55                  60

Asp Arg Ala Thr Gly Leu His Tyr Gly Ile Ile Ser Cys Glu Gly Cys
65                  70                  75                  80

Lys Gly Phe Phe Lys Arg Ser Ile Cys Asn Lys Arg Val Tyr Arg Cys
                85                  90                  95

Ser Arg Asp Lys Asn Cys Val Met Ser Arg Lys Gln Arg Asn Arg Cys
            100                 105                 110

Gln Tyr Cys Arg Leu Leu Lys Cys Leu Gln Met Gly Met Asn Arg Lys
        115                 120                 125

Ala Ile Arg Glu Asp Gly Met Pro Gly Gly Arg Asn Lys Ser Ile Gly
    130                 135                 140

Pro Val Gln Ile Ser Glu Glu Glu Ile Glu Arg Ile Met Ser Gly Gln
145                 150                 155                 160

Glu Phe Glu Glu Glu Ala Asn His Trp Ser Asn His Gly Asp Ser Asp
                165                 170                 175

His Ser Ser Pro Gly Asn Arg Ala Ser Glu Ser Asn Gln Pro Ser Pro
            180                 185                 190

Gly Ser Thr Leu Ser Ser Ser Arg Ser Val Glu Leu Asn Gly Phe Met
        195                 200                 205

Ala Phe Arg Glu Gln Tyr Met Gly Met Ser Val Pro Pro His Tyr Gln
    210                 215                 220

Tyr Ile Pro His Leu Phe Ser Tyr Ser Gly His Ser Pro Leu Leu Pro
225                 230                 235                 240

Gln Gln Ala Arg Ser Leu Asp Pro Gln Ser Tyr Ser Leu Ile His Gln
                245                 250                 255

Leu Leu Ser Ala Glu Asp Leu Glu Pro Leu Gly Thr Pro Met Leu Ile
            260                 265                 270

Glu Asp Gly Tyr Ala Val Thr Gln Ala Glu Leu Phe Ala Leu Leu Cys
        275                 280                 285

Arg Leu Ala Asp Glu Leu Leu Phe Arg Gln Ile Ala Trp Ile Lys Lys
    290                 295                 300

Leu Pro Phe Phe Cys Glu Leu Ser Ile Lys Asp Tyr Thr Cys Leu Leu
305                 310                 315                 320

Ser Ser Thr Trp Gln Glu Leu Ile Leu Leu Ser Ser Leu Thr Val Tyr
                325                 330                 335

Ser Lys Gln Ile Phe Gly Glu Leu Ala Asp Val Thr Ala Lys Tyr Ser
            340                 345                 350

Pro Ser Asp Glu Glu Leu His Arg Phe Ser Asp Glu Gly Met Glu Val
        355                 360                 365

Ile Glu Arg Leu Ile Tyr Leu Tyr His Lys Phe His Gln Leu Lys Val
    370                 375                 380

Ser Asn Glu Glu Tyr Ala Cys Met Lys Ala Ile Asn Phe Leu Asn Gln
385                 390                 395                 400

Asp Ile Arg Gly Leu Thr Ser Ala Ser Gln Leu Glu Gln Leu Asn Lys
                405                 410                 415

Arg Tyr Trp Tyr Ile Cys Gln Asp Phe Thr Glu Tyr Lys Tyr Thr His
            420                 425                 430

Gln Pro Asn Arg Phe Pro Asp Leu Met Met Cys Leu Pro Glu Ile Arg
        435                 440                 445

Tyr Ile Ala Gly Lys Met Val Asn Val Pro Leu Glu Gln Leu Pro Leu

450                 455                 460

Leu Phe Lys Val Val Leu His Ser Cys Lys Thr Ser Val Gly Lys Glu
465                 470                 475                 480

Ser Thr Ala Pro Pro Thr Asp Val Ser Leu Gly Asp Glu Leu His Leu
                485                 490                 495

Asp Gly Glu Asp Val Ala Met Ala His Ala Asp Ala Leu Asp Gly Phe
            500                 505                 510

Asp Leu Asp Met Leu Gly Asp Gly Asp Ser Pro Gly Pro Gly Phe Thr
        515                 520                 525

Pro His Asp Ser Ala Pro Tyr Gly Ala Leu Asp Met Ala Asp Phe Glu
    530                 535                 540

Phe Glu Gln Met Phe Thr Asp Ala Leu Gly Ile Asp Glu Leu Lys Ala
545                 550                 555                 560

Cys Met Pro Ala Gly Arg Leu
                565

<210>  32
<211>  14
<212>  RNA
<213>  Artificial Sequence

<220>
<223>  complementary sequence

<400>  32
guagccuuga ccuc                                                         14

<210>  33
<211>  90
<212>  RNA
<213>  Artificial Sequence

<220>
<223>  guideRNA

<400>  33
guagccuuga ccucguuuua gagcuagaaa uagcaaguua aaauaaggcu aguccguuau       60

caacuugaaa aaguggcacc gagucggugc                                        90

<210>  34
<211>  18
<212>  DNA
<213>  Artificial Sequence

<220>

<223>  consensus sequence

<220>
<221>  N
<222>  (1)..(1)
<223>  N1 is selected from A, T, G and C

<220>
<221>  misc_feature
<222>  (1)..(7)
<223>  n is a, c, g, or t

<220>
<221>  N
<222>  (2)..(2)
<223>  N2 is selected from A, T, G and C, and preferably is T

<220>
<221>  N
<222>  (3)..(3)
<223>  N3 is selected from A, T, G and C, and preferably is C

<220>
<221>  N
<222>  (4)..(4)
<223>  N4 is selected from A, T, G and C, and preferably is A

<220>
<221>  N
<222>  (5)..(5)
<223>  N5 is selected from A, T, G and C, and preferably is A

<220>
<221>  N
<222>  (6)..(6)
<223>  N6 is selected from A, T, G and C, and preferably is G

<220>
<221>  N
<222>  (7)..(7)
<223>  N7 is selected from A, T, G and C, and preferably is T or G, more
       preferably is T

<220>
<221>  N
<222>  (11)..(11)
<223>  N8 is selected from A, T, G and C, and preferably is A

<220>
<221>  misc_feature
<222>  (11)..(18)
<223>  n is a, c, g, or t

<220>
<221>  N
<222>  (12)..(12)
<223>  N9 is selected from A, T, G and C, and preferably is G or T, more
       preferably is G

<220>
<221>  N
<222>  (13)..(13)

<223>  N10 is selected from A, T, G and C, and preferably is T or G

<220>
<221>  N
<222>  (14)..(14)
<223>  N11 is selected from A, T, G and C, and preferably is T

<220>
<221>  N
<222>  (15)..(15)
<223>  N12 is selected from A, T, G and C, and preferably is C

<220>
<221>  N
<222>  (16)..(16)
<223>  N13 is selected from A, T, G and C, and preferably is A

<220>
<221>  N
<222>  (17)..(17)
<223>  N14 is selected from A, T, G and C

<220>
<221>  N
<222>  (18)..(18)
<223>  N15 is selected from A, T, G and C

<400>  34
nnnnnnntca nnnnnnnn                                                    18